# EUROPEAN PATENT SPECIFICATION

(11) **EP 0 894 043 B1**
(45) Date of publication and mention of the grant of the patent: **31.10.2001**
(21) Application number: 97915950.6
(22) Date of filing: 14.03.1997
(51) Int. Cl.: B29C 37/00, B29C 39/24

(54) **METHOD OF FORMING ARTICLES AND PATTERNING SURFACES VIA CAPILLARY MICROMOLDING**
VERFAHREN ZUM FORMEN VON GEGENSTÄNDEN UND ZUM MIKROSTRUKTURIEREN VON OBERFLÄCHEN DURCH GIESSFORMEN MIT KAPILLARWIRKUNG
PROCEDE DE FORMATION D'ARTICLES ET DE SURFACES A MOTIFS PAR MICROMOULAGE CAPILLAIRE

(30) Priority: 15.03.1996 US 616929 P
(43) Date of publication of application: 03.02.1999
(73) Proprietor: PRESIDENT AND FELLOWS OF HARVARD COLLEGE, Cambridge, Massachusetts 02138-5701 (US)
(72) Inventor: KIM, Enoch, Boston, MA 02116 (US); XIA, Younan, Cambridge, MA 02138 (US); MRKSICH, Milan, Chicago, IL 60637 (US); JACKMAN, Rebecca, Jane, Boston, MA 02114 (US); WHITESIDES, George, M., Newton, MA 02158 (US)
(74) Representative: Jump, Timothy John Simon
(86) International application number: US9704005
(87) International publication number: WO9733737

(56) References cited:
- EP-A- 0 112 721
- EP-A- 0 672 765
- WO-A-96/29629
- US-A- 4 258 001
- US-A- 4 555 414
- US-A- 5 227 474
- US-A- 5 345 869
- US-A- 5 385 116
- US-A- 5 439 829
- US-A- 5 512 131
- US-A- 5 620 850
- ADVANCED MATERIALS, vol. 8, no. 3, 1 March 1996, WEINHEIM, DE, pages 245-247, XP000558255 KIM E ET AL: "TWO- AND THREE-DIMENSIONAL CRYSTALLIZATION OF POLYMERIC MICROSPHERES BY MICROMOLDING IN CAPILLARIES"
- PATENT ABSTRACTS OF JAPAN vol. 096, no. 001, 31 January 1996 & JP 07 237229 A (CANON INC), 12 September 1995,
- APPLIED PHYSICS LETTERS, vol. 68, no. 7, 12 February 1996, WOODBURY, NY, USA, pages 1022-1024, XP000559984 JOHN ST P M ET AL: "MICROCONTACT PRINTING AND PATTERN TRANSFER USING TRICHLOROSILANES ON OXIDE SUBSTRATES"
- JOURNAL OF THE ELECTROCHEMICAL SOCIETY, vol. 142, no. 2, February 1995, pages 628-633, XP002015603 KIM E ET AL: "COMBINING PATTERNED SELF-ASSEMBLED MONOLAYERS OF ALKANETHIOLOTES ON GOLD WITH ANISOTROPIC ETCHING OF SILICON TO GENERATE CONTROLLED SURFACE MORPHOLOGIES"

## Description

The present invention relates to a method of creating a pattern of a species according to the precharacterising part of claim 1 and to a method of creating a pattern of a species according to the precharacterising part of claim 21.

These methods concern generally microprocesses at surfaces, and more particularly the formation of micropatterned articles on surfaces and mechanisms for micro-scale positioning of biologically active agents at predetermined regions of a surface.

In the fields of chemistry, biology, materials science, microelectronics, and optics, the development of devices that are small relative to the state of the art and conveniently and relatively inexpensively reproduced is important.

A well-known method of production of devices, especially in the area of microelectronics, is photolithography. According to this technique, a negative or positive resist (photoresist) is coated onto an exposed surface of an article. The resist then is irradiated in a predetermined pattern, and portions of the resist that are irradiated (positive resist) or nonirradiated (negative resist) are removed from the surface to produce a predetermined pattern of resist on the surface. This is followed by one or more procedures. According to one, the resist may serve as a mask in an etching process in which areas of the material not covered by the resist are chemically removed, followed by removal of resist to expose a predetermined pattern of a conducting, insulating, or semiconducting material. According to another, the patterned surface is exposed to a plating medium or to metal deposition (for example under vacuum) followed by removal of resist, resulting in a predetermined plated pattern on the surface of the material. In addition to photolithography. x-ray and electron-beam lithography have found analogous use.

In an article entitled "Materials for Optical Data Storage", by Emmelius, et al., *Angewandte Chemie, Int. Ed. (English),* 28, 11, 1445-1600 (November, 1989), a review of methods of making CD/ROM, WORM, and EDRAW optical storage disks is presented. According to one method, photolithography is used to create a pattern of protrusions on a surface that can serve as a master for fabrication of articles that have a surface including a series of ridges and protrusions complementary to the photolithographically-produced master. These articles, including microridges and grooves at one surface, can be combined with other materials in a layered structure to form an optical storage device. An article in the Phillips Technical Review, volume 40, number 10 (1982), entitled "Manufacture of LaserVision Video Disks by a Photopolymerization Process", by Haverkorn, et al., discusses similar technology. U.S. Patent Nos. 5,170,461 (Yoon, et al.), 4,959,252 (Bonnebat. et al.) and 5,141,785 (Yoshinada, et al.) describe optical elements such as waveguides and optical recording media. Yoshinada, et al. describe a process involving coating a substrate with a polymer or prepolymer, pressing a contoured stamp into the polymer or prepolymer to create a contoured pattern in a surface of the polymer or prepolymer complementary to the contoured surface of the stamp, removing the stamp, and adding a reflective layer to the contoured surface of the polymer or prepolymer for use as an optical device.

Photolithographic techniques for fabricating surfaces with positional control of chemical functionalities at submicron resolution is described in an article entitled "Patterning of Self-Assembled Films Using Lithographic Exposure Tools", by Dressick. et al., *Jpn. J. Appl. Phys.,* 32, 5829-5839 (December, 1993). The technique involves exposure of a self-assembled film to deep UV irradiation through a mask. According to one technique, photochemical cleavage of an organic group occurs in exposed regions followed by chemical reactivity selectively at those regions.

Photolithography has found application in the biological arena as well. Sundberg, et al. describe a method for patterning receptors, antibodies, and other macromolecules at precise locations on solid substrates using photolithographic techniques in combination with avidin or streptavidin/biotin interaction in an article entitled "Spatially-Addressable Immobilization of Macromolecules on Solid Supports", *J. Am. Chem. Soc*., 117, 12050-12057 (1995).

Biological and chemical interactions can be studied on the micro scale using combinatorial chemistry. This technique, as described in *Chemical & Engineering News*, 74, 7, 28-73 (February 12, 1996), involves formation of different biological or chemical species in a grid pattern on a surface and used, for example, to screen compounds for potential biological or chemical activity. An article entitled "Combinatorial Chemistry―Applications of Light-Directed Chemical Synthesis", by Jacobs, et al., *Trends in Biotechnology.* 12, 19-26 (January. 1994) describes a photolithographic process used in a spatially-addressable synthesis technique for forming a combinatorial library involving photolithography. A surface is derivatized with amine linkers that are blocked by a photochemically cleavable protecting group. The surface is selectively irradiated to liberate free amines that can be coupled to photochemically blocked building blocks. The process is repeated with different regions of the surface being exposed to light and involved in synthesis until a desired array of different compounds, in a grid pattern on the surface, is prepared. Each of these compounds then is assayed simultaneously for binding or activity. Binding "hits" can be identified by the particular location at which binding on the surface occurs.

Other techniques for forming features on surfaces are known. John, et al. in "Microcontact printing and pattern transfer using trichlorosilanes on oxide substrates". *Appl. Phys. Lett*., 7, February 12, 1996, pages 1022-1024, describe using an elastomeric stamp for microcontact printing to pattern silicon, aluminum, and titanium substrates using octadecyltrichlorosilane as ink. Patterns were transferring into the substrates using both dry and wet etching. Aluminum and titanium were etched using an electron cyclotron resonance plasma source at high ion energies and low pressure. Kim, et al., "Combining patterned self-assembled monolayers of alkanethioletes on gold with anisotropic etching of silicon to generate controlled surface morphologies", *J. Electrochem. Soc.,* 142, 2, February 1995, describe a technique involving patterning self-assembled monolayers of hexadecanethiolate onto gold films supported on titanium-primed silicon wafers using microntact printing. Wet etching of the gold, and subsequently of the silicon, using the three systems of resists based on patterned self-assembled monolayers, produced features in silicon having dimensions in the 1 to 10 microns scale. U.S. Patent No. 4,555,414 (Hoover, et al.) describe a multilayered composite product including a base, a patterned cured resin bonded to the base, and an applied metal layer selectively bonded to the patterned cured resin. The product is useful as an electrical circuit.

An article entitled "Microcontact Printing and Pattern Transfer Using Trichlorosilanes on Oxide Substrates", describes patterning on a substrate with an elastomeric stamp, taking advantage of bonding between trichlorosilanes and oxides. *Appl. Phys. Lett.,* Volume 68, 1022-1024 (1996). An article entitled "Combining Patterned Self-Assembled Monolayers of Alkanethiolates on Gold with Anisotropic Etching of Silicon to Generate Controlled Surface Morphologies" describes the use of patterning self-assembled monolayers of hexadecanethiolate onto gold films. *J. Electrochem. Soc*., Volume 142, 628-633 (1995). U.S. Patent no. 5,345,869 (Treverton et al, September 13 1994) describes a method for preparing lithographic plates comprising a substrate carrying an oxide layer derived from a Type A sol. European Patent Application 672 765 A1 (Reetz, published September 20, 1995) describes electrochemical reduction of metal salts to prepare metal colloids having particle sizes of less than 30 nm. U.S. Patent No. 5,439,829 (Anderson et al., August 8, 1995) describes the use of a chelating agent for covalently bonding a biologically active molecule to a support containing a chelated transition metal ion. U.S. Patent No. 5,227,474 (Johnson et al., July 13, 1993) describes the use of a bifunctional chelating agent for binding metal ions to biologically active molecules. US. Patent No. 4,555,414 (Hoover et al., November 26, 1985) describes the use of a multi-layered composite product having a patterned cured resin bound to a base and a metal layer selectively bonded to the patterned cured resin. U.S. Patent No. 5,620,850 (Bamdad et al., April 15, 1993) described chelating agents on surfaces.

These and other techniques for modifying a surface on the micro scale typically require relatively sophisticated apparatus, are expensive, and generally consume more reactants and produce more by-products in collateral fabrication steps than is optimal. Accordingly, it is an object of the invention to provide a variety of techniques for modifying a surface chemically and/or biologically at the micro and nanoscale, conveniently, inexpensively, and reproducibly. In particular, it is also an object of the invention to provide a technique for creating a pattern of a species on at least one region of a substrate surface.

The present invention provides techniques for derivatizing surfaces, biologically, chemically, or physically, according to predetermined patterns. The derivatized surfaces find a variety of uses in a variety of technical areas, or a structure formed on the surface can be removed from the surfaces and find utility separate from the surface.

In a first aspect of the invention, there is provided a method of creating a pattern of a species at a first region proximate a substrate surface, the pattern including a portion having a lateral dimension of less than 1 mm, while leaving a second region proximate the substrate surface, contiguous with the first region, free of the species, using an article having a contoured surface including at least one indentation defining a pattern, characterized in that the method involves creating the pattern of the species at the first region proximate the substrate surface in a manner that corresponds to and is in register with the indentation pattern of the article.

In a second aspect of the invention , there is provided a method of creating a pattern of a species comprising providing a surface carrying an essentially even distribution thereacross of chelating agents coordinating metal ions, characterized in that method involves applying to two discrete regions and at the surface a species that is a biologically active agent, while leaving a region intervening the two discrete regions free of the biologically active agent.

Preferred embodiments of the invention in either of its aspects are defined in the sub-claims and described below.
Fig. 1 illustrates schematically an arrangement for derivatizing a surface in a predetermined pattern according to one embodiment of the invention;
Fig. 2 is a schematic illustration of a technique for transferring a chemically or biochemically active agent or fluid precursor of an article from essentially linear indentations of an applicator defining an indentation pattern to a substrate surface in a pattern corresponding to the indentation pattern;
Figs. 3a-d are photocopies of scanning electron micrographs (SEMs) of patterned polymeric structures formed from hardenable fluid precursors in which the patterned structures remain at the surface (Figs. 3a-c) or are removed from the surface to form a free-standing structure (Fig. 3d);
Figs. 4a-h are photocopies of SEM images of inorganic and organic microstructures patterned on surfaces in accordance with the invention;
Fig. 5 is a photocopy of an electron micrograph of microspheres assembled in a predetermined pattern proximate a substrate surface from a fluid precursor in accordance with the invention;
Figs. 6a-c are photocopies of SEM images of metallic microstructures formed proximate predetermined regions of a substrate surface in accordance with the invention;
Figs. 7a-c are photocopies of SEM images of a substrate surface derivatized in a pattern with resist followed by lithography to etch the substrate surface in a pattern complementary to the resist pattern;
Fig. 8 illustrates schematically the formation of a free-standing article from a fluid precursor, using a substrate surface and a forming article including a pattern of indentations in accordance with the invention;
Figs. 9a-d are photocopies of SEM images of a free-standing article prepared in accordance with the technique schematically illustrated in Fig. 8 and use of that article as a mask adjacent a substrate surface in vapor deposition of metal (Fig. 9b) or creation of a secondary resist formed by a self-assembled monolayer deposited in a pattern complementary to that of the mask, followed by removal of the mask and vapor deposition of metal in a pattern complementary to the secondary resist pattern (Figs. 9c-d);
Figs. 10a-c illustrate schematically (Fig. 10a), and via photoreproduction of optical micrographs (Fig. 10b, c), a process involving derivatizing a surface with resist via micromolding, a mask so produced, and a substrate surface etched selectively at regions not covered by the mask;
Fig. 11 illustrates schematically a technique for transfer of a chemically or biochemically active agent or a fluid precursor of an article from an applicator having a discontinuous indentation pattern to regions proximate a substrate surface in a pattern corresponding to the indentation pattern;
Fig. 12 illustrates schematically the transfer, from an article including an indentation pattern, of a chemically or biochemically active agent or other fluid species to a nonplanar substrate surface in a pattern corresponding to the indentation pattern;
Fig. 13 illustrates schematically a multi-layered article formed using successive micromolding techniques of the invention that can serve as a waveguide; and
Figs. 14a-k illustrate schematically the creation of a combinatorial library in accordance with the invention.

The present invention provides techniques for placement, at regions proximate a substrate surface, of chemically or biochemically active agents, fluid precursors of articles to be immobilized proximate a substrate surface, and/or other species desirably transferred to a region or regions proximate a substrate surface in a pattern. The invention utilizes an applicator having a pattern of indentations that can be used to transfer such a species from the indentations to a region proximate the substrate surface or that can serve as a mold that when, positioned proximate a substrate surface, can define a region in which such a species is positioned.

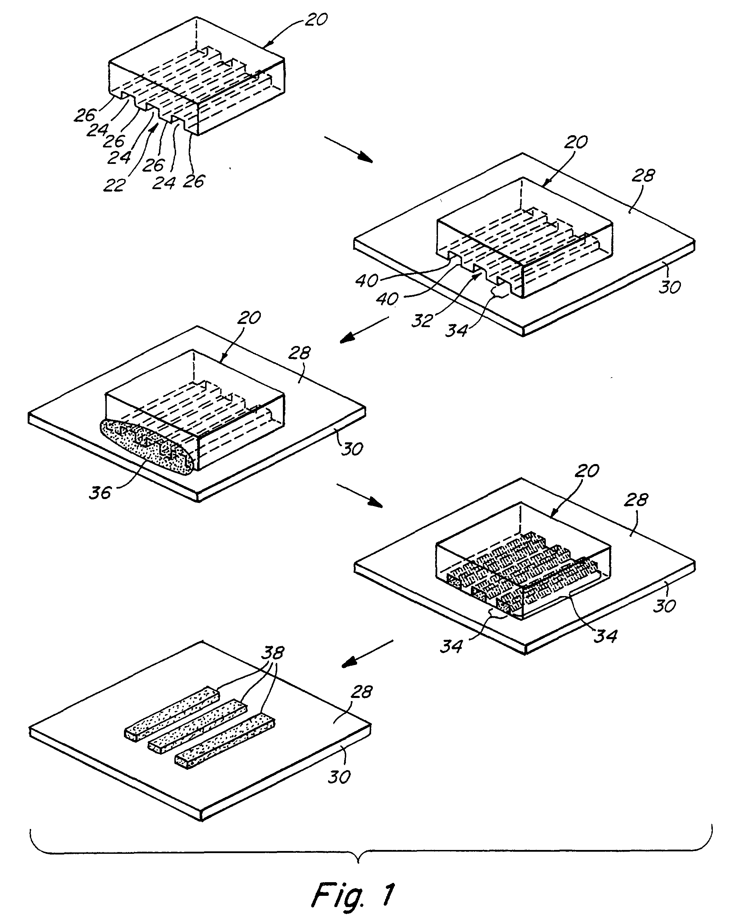

Fig. 1 illustrates schematically a technique for derivatizing a substrate surface according to a pattern of, for example, a polymeric article, a pattern of microbeads optionally carrying a chemical or biochemically active species, a catalyst or other activating agent for promoting a chemical reaction such as metal plating at the surface, a fluid carrying a dissolved or suspended species to be deposited or precipitated, or the like. For purposes of illustration, the procedure schematically illustrated in Fig. 1 will be described with respect to a hardenable prepolymeric fluid that is hardened at the surface to form a patterned polymeric article. An article 20 includes an application surface 22 having formed therein a plurality of indentations 24 that together define a linear, patterned array of indentations 24 that are contiguous with a contact surface 26. Article 20 according to one embodiment, is an applicator used to transfer a species, in a pattern, to a region or regions proximate the substrate surface, or a forming article or micromold placed proximate a substrate surface and used to guide a fluid species so as to position the species in a pattern at a predetermined region or regions proximate the substrate surface. As used herein, the term "proximate" is meant to define at a substrate surface, that is, in contact with a substrate surface, or at a position near a substrate surface and fixed relative to the substrate surface. For example, if a substrate surface carries an adhesion promoter, for example a self-assembled monolayer, activity at the surface of the self-assembled monolayer is intended to mean activity proximate the substrate surface. When forming article 20 is placed proximate a surface 28 of a substrate 30, contact surface 26 of the article seals portions of surface 28 that it contacts, thereby forming channels 32 defined by indentations 24 and portions 34 of substrate surface 28 not contacted by contact surface 26. In this manner a micromold is created, which is defined by article 20 and substrate surface 28.

A fluid 36 that, according to the embodiment illustrated, is a precursor of a patterned, polymeric structure (but can be one of a variety of species such as a carrier of a chemically or biochemically active agent, etc., as described herein) is placed adjacent one or more openings of channels 32 and introduced into the channels and allowed to flow adjacent portions 34 of substrate surface 28 in register with indentations 24. Fluid precursor 36 can be urged to flow via, for example, pressure applied to the fluid as it is positioned so as to enter the channels, or vacuum created within the channels by, for example, connection of the outlets of the channels to a source of vacuum. Alternatively, according to one aspect of the invention, the fluid can be allowed to flow into the mold via capillary action. Capillary filling of the mold is especially useful when the mold is of very small dimension (in particular in the micro scale) and is defined herein to mean that when a fluid precursor is positioned adjacent an opening or channel 32 formed by a portion 34 of the substrate surface and an indentation 24 of article 20, the fluid precursor will flow into at least a portion of the channel spontaneously.

Subsequent to introduction of the fluid precursor into the mold defined by channels 32, the fluid precursor can be hardened before or after removal of applicator 20 from substrate surface 28 (or where the fluid is a carrier of a species to be deposited or precipitated, the fluid can dissipate, i.e., evaporate, be absorbed into applicator 20, or the like). Where the fluid is viscous enough, or is allowed to reach a particular level of viscosity, the applicator can be removed and the precursor hardened at the surface without unacceptable loss of dimensional integrity.

The fluid precursor can be a solution of monomer in a fluid carrier and is polymerized at the surface with article 20 in place. Article 20 then is removed. A structural article 38, in a pattern corresponding to the indentation or mold pattern 24 of article 20, results on substrate surface 28 from the described procedure. According to the description of the process illustrated in Fig. 1, structure 38 is a polymeric structure formed from a fluid prepolymeric precursor.

Where the structure 38 formed according to this embodiment is a polymeric structure, it can be thermally polymerized on substrate surface 28 via application of heat to the substrate and/or article 20 or, if article 20 is removed prior to polymerization, via convective or radiative heat; photopolymerized if substrate 30 and/or article 20 are transparent to radiation, or subsequent to removal of article 20. Free-radical polymerization can be carried out as well. These and other forms of polymerization are known to those of ordinary skill in the art and can be applied to the techniques of the present invention without undue experimentation. All types of polymerization, including cationic, anionic, copolymerization, chain copolymerization, cross-linking, and the like can be employed, and essentially any type of polymer or copolymer formable from a fluid precursor can be patterned on surface 28 in accordance with the invention. An exemplary, non-limiting list of polymers that are suitable include urethane polymers, polyamides, polycarbonates, polyacetylenes and polydiacetylenes, polyphosphazenes, polysiloxanes, polyolefins, polyesters, polyethers, poly(ether ketones), poly(alkylene oxides), poly(ethylene terephthalate), poly(methyl methacrylate), polystyrene, and derivatives and block, random, radial, linear, or teleblock copolymers, cross-linkable materials such as proteinaceous material and/or blends of the above. Gels are suitable where dimensionally stable enough to maintain structural integrity upon removal of article 20 from substrate surface 28. The particular polymer, copolymer, blend, or gel selected is not critical to the invention, and those of skill in the art can tailor a particular material for any of a wide variety of applications.

According to one embodiment, a polymerizable or cross-linkable species (optionally in a fluid carrier) including an admixed biochemically active agent such as a protein can be made to form a pattern on substrate surface 28 according to the described technique. For example, carboxylated Dextran™ can carry admixed protein, be introduced into channels 34, and hardened to form articles 38. Where the Dextran™ carries admixed biologically active agent, the article can be exposed to a medium suspected of containing a biological binding partner of the biochemical agent, and any biochemical binding or other interaction detected via, for example, diffraction. That is, where article 38 defines diffraction grating, the degree of diffraction can be affected by biological binding between the biological agent compounded within article 38 and an analyte that is a biological binding partner of the compounded agent. Determination of a change in diffraction at surface 28 is indicative of the presence of analyte in the medium brought into contact with article 38. According to another embodiment, a species such as polymerizable or cross-linkable species can entirely coat surface 28, article 20 can be placed adjacent surface 28, a biological agent can be introduced into channels 34 and allowed to admix with the polymerizable or cross-linkable species, and prior to or subsequent to removal of article 20 species on surface 28 can be polymerized or cross-linked. In this manner, a surface having a pattern of biological agent compounded therein is produced, and can serve as a sensor for a biological binding partner of the biological agent via change in refraction or diffraction of light at the surface.

Where electrical conductivity is desired, an electrically-conductive polymer can be selected, and this can have significant application in the microelectronics industry, as would be recognized by one of ordinary skill in the art.

The invention is intended to encompass creation of a wide variety of structures or patterns of species on substrate surfaces from fluid precursors. The precursor can be any fluid that can flow into the mold defined by indentations 24 and portions 34 of substrate surface 28, and those of ordinary skill in the art can determine, without undue experimentation, which fluids will readily flow into such a mold based upon dimension of the mold and viscosity of the fluid. In most instances, the viscosity of the fluid can be adjusted, for example by diluting or concentrating the fluid, to achieve a level of viscosity suitable for flow into the mold at a desired rate. The polarity of the fluid can be tailored as well, with reference to the chemical characteristic of the substrate surface or micromold, to facilitate fluid carrier flow.

Alternatively, the patterned article 38 is not a polymer or cross-linked organic species as described above, but is a non-polymerized organic species that is dissolved or dispersed in a fluid carrier to form fluid precursor 36 which is introduced into mold channels 32, whereupon the fluid carrier or solvent dissipates (e.g.. is removed via evaporation from the mold channels and/or absorption into the substrate or applicator 20). According to yet another embodiment, patterned structure 38 is an inorganic structure, such as a salt or ceramic. A salt soluble in a fluid precursor can be prepared as a solution 36 defining a fluid precursor that is introduced into mold channels 32 and precipitated as a patterned salt structure 38 by removal of solvent via evaporation, adsorption, or other physical or chemical change to the surrounding environment. Inorganic salts or ceramics can be carried as a suspension in a fluid carrier, flowed into channels 32, and precipitated or deposited. Metals, such as those commonly deposited from pastes in accordance with thick-film silk-screening techniques, can be applied to defined regions of substrate surface 28 where a paste is sufficiently fluid, or the paste and/or metal can be carried in a fluid as a suspension or sol in fluid precursor 36. Those of ordinary skill in the art will recognize that a wide variety of non-electrically conductive, electrically semi-conductive, and electrically-conductive structures can be patterned proximate a substrate surface according to the inventive technique. These structures can be deposited in any pattern that corresponds to an indentation pattern formable in an applicator or micromold 20 and can include lateral dimensions through a wide range as described below.

Further, a biologically active agent can be dissolved or suspended in a fluid carrier as a fluid precursor 36 and introduced into channels 32 adjacent portions 34 of surface 28 and, prior or subsequent to removal of micromold 20, allowed to engage in a biochemical interaction proximate regions 34 of substrate surface 28. For example, a biochemical agent can include a biotin linker while substrate surface 28 carries immobilized avidin, and biochemical interaction can be allowed to take place at regions 34 of substrate surface 28 in this manner, linking the biochemical agent to the substrate surface at regions 34. Biochemical agents can be immobilized proximate regions 34 of the substrate surface according to other techniques as well. For example, where substrate surface 28 exposes a hydrophobic functionality, a biological agent such as a protein can be non-covalently immobilized at regions 34 of the substrate surface. To control orientation of a protein or other biochemical agent immobilized at a substrate surface via hydrophobic interaction, a hydrophobic chemical moiety can be coupled to the biochemical agent at a region of the agent remote from its active site. In this manner, the agent can be hydrophobically coupled to the surface and maintain exposure, away from the surface, of its biochemically active region. One of ordinary skill in the art can conduct a simple test to determine whether a biochemical agent is suitable for use with the described technique. The binding constant of a candidate biochemical agent for a target species can be determined using standard ELISA techniques. Then, the candidate biochemical agent can be hydrophobically immobilized (or immobilized in any other manner described herein or known to those of ordinary skill in the art, for example via a polyamino acid tag coupled to a metal ion immobilized at the surface by a chelating agent) at a variety of surfaces, and then assays can be performed to determine whether the agent has retained its ability to biologically bind to the target species or has been denatured and is unable to bind (this exemplary test is particularly useful in connection with biological agents that, in their native form only, bind target species, but when denatured do not bind the target species).

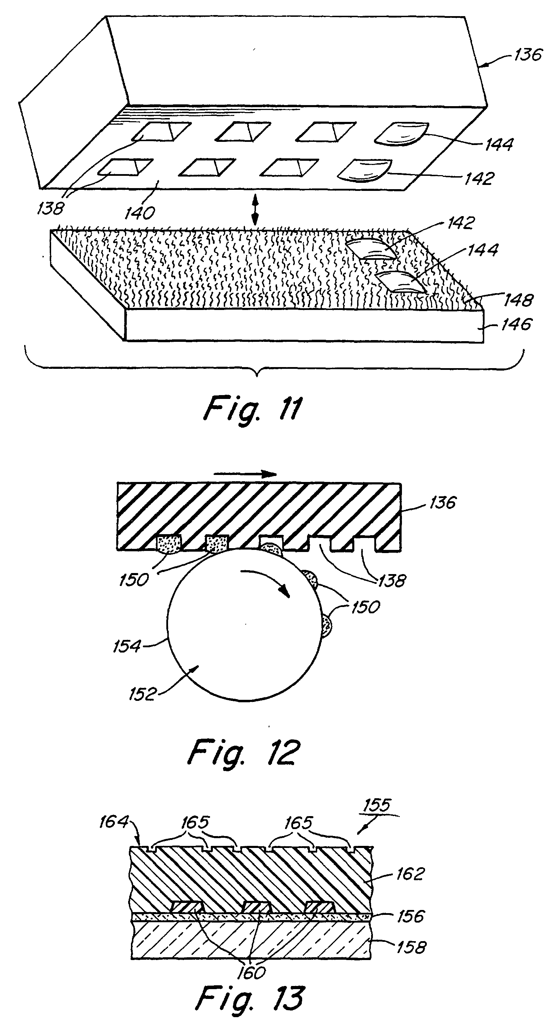

Biochemical recognition can be exploited in immobilization of a particular biochemical agent desirably patterned on substrate surface 28. For example, a first agent can be immobilized (for example using hydrophobic coupling) at regions 38 of the substrate surface, and a second agent (which is a biological binding partner of the first agent) then can be immobilized at regions 34. The second step in which the desired agent is immobilized at regions 34 can be carried out with or without micromold 20 proximate the substrate surface. Biochemical recognition involving partners also can be exploited to trap biological agents at regions 34 of the substrate surface using other biological agents that have been immobilized at regions 34. Biochemical recognition involving partners such as antibody/antigen, antibody/hapten, enzyme/substrate, enzyme/inhibitor, enzyme/cofactor, biotin/avidin, binding protein/substrate, carrier protein/substrate, lectin/carbohydrate, receptor/hormone, receptor/effector, complementary strands of nucleic acid, repressor/inducer, and the like can be exploited in connection with the technique. Those of ordinary skill will recognize a variety of uses for placement of such biochemically active agents at predetermined portions of a substrate surface in a pattern, for example as discussed below with reference to Fig. 13 and as disclosed in co-pending, commonly-owned U.S. Patent No. 5,512,131 of Kumar, et al. and International Patent Application Publication No. WO 96/29629.

According to embodiments in which the biochemical activity of a biologically active agent can be detrimentally affected by environmental factors, a fluid carrier of the biologically active agent should be selected so as not to detrimentally affect the biochemical activity of the agent. For example, if a protein is to be patterned on the surface and used in an interaction that cannot take place (or takes place at an unacceptably low level) when the protein is denatured, a fluid carrier should be selected that does not denature the protein or otherwise detrimentally affect the biological binding interaction of the protein that is to be exploited. Additionally, a micromold 20 should be selected and/or fabricated in a manner such that the surfaces of indentations 24 that can come into contact with a biologically active agent will not detrimentally affect the performance of the agent. For example, if micromold 20 is fabricated from a material that could denature a protein, then if used with the protein the interior surfaces of indentations 24 can be chemically altered, for example via grafting with polyethylene glycol, to render the surfaces non-destructive of the agent.

According to yet another alternative, fluid precursor 36 carries a suspended or dissolved chemically active agent that is an activating agent as described in a co-pending, commonly owned U.S. application serial no. 08/616,692 of Hidber, et al, entitled "Microcontact Printing of Catalytic Colloids", referenced above. When a fluid carrier is used in this and other embodiments, it can form part of a species or article immobilized proximate the substrate surface or can dissipate, for example via evaporation or adsorption into the applicator or substrate surface, leaving the species carried in the fluid carrier immobilized at the surface.

A non-limiting list of chemically active agents that can be patterned on a surface in accordance with the invention includes agents as described by Lando (U.S. Patent Nos. 3,873,359; 3,873,360; and 3,900,614) which can render a substrate surface amenable to metal plating, catalytic activating agents such as finely distributed metal particles and clusters such as conventional metal powders, substrate-fixed metal clusters or multimetallic clusters that are well known as valuable heterogeneous and homogeneous catalysts in organic chemistry, inorganic chemistry, and electrochemistry, etc. With reference to the application of Hidber, et al., such agents can include those capable of being carried by an applicator, transferred from the applicator to a surface in a form in which it can effect a chemical reaction (such as a metal deposition reaction), and immobilized at the surface with a degree of adhesion and for a period of time sufficient to participate in the desired chemical reaction. As such. one class of activating agents provided in accordance with the invention are distinguished from prior art agents applied with an applicator such as a stamp, for example as disclosed by Lando (U.S. Patent Nos. 3,873,359, 3,873,360, and 3,900,614), in that the activating agent of the present invention is in a form suitable for effecting reaction such as metal plating or catalytic action when transferred to the surface. According to preferred embodiments, further chemical reaction at the surface to convert a precursor to a suitable agent, as necessitated in the referenced prior art methods, is not required.

Metal deposition reactions contemplated include electrochemical deposition and electroless deposition, generally involving reduction of a metal cation to create the metal, facilitated in part by the lowering of the electrochemical potential involved in the deposition.

Activating agents that are finely distributed metal particles and clusters, such as conventional metal powders, including substrate-fixed metal clusters or multimetallic clusters are suitable for use as activating agents in accordance with the invention, and are well known as valuable heterogeneous and homogeneous catalysts in organic, inorganic, and electrochemistry. Exemplary activating agents include one or more metals of periodic table groups Ib, IIb, III, IV, V, VI. Vllb. VIII, lanthanides, and actinides, preferably copper and any metal more noble than copper, in particular Pd, Au, Ag, Pt, and Cu. Hydrogenation catalysts for example those effective in hydrogenating olefins or aromatics, as in the partial hydrogenation of benzene to form cyclohexene, with a substrate-fixed ruthenium activating agent or bimetallic activating agent (e.g. Ru/Sn) are contemplated. Zirconium and titanium catalysts, among others, are suitable for use in the invention that catalyze polymerization, such as polymerization of olefins such as ethylene, and these are intended to form part of the invention. Other examples of catalytic activating agents include those used in Heck reactions, e.g. in the Pd-catalyzed reaction of bromobenzene and styrene to form stilbene. Activating agents that are heterogeneous catalysts are also useful as electrocatalysts in fuel cells (in particular substrate-fixed Pt and Pt/Ru clusters). Activating agents prepared according to the invention can be homogeneous catalysts, such as those used in two phase systems (for instance H₂O/toluene), such as e.g. betaine-stabilized Pd clusters soluble in H₂O. Activating agents that are embedded in polymers can be used to prepare materials for electronic, optical and magnetic applications. Suitable embedding polymers include organic polymers, such as poly-p-phenylene-vinylene, polymethyl methacrylate, polysilanes, and polystyrene, or inorganic polymers, such as zeolites, silicates, and metal oxides. The well-known sol-gel process can be used to incorporate metal clusters in amorphous metal oxide materials (e.g. SiO₂) as activating agents.

Soluble metal clusters that are activating agents can also be surface-deposited to prepare novel materials for applications in optics and electronics, e.g. Pd on HOPG (highly oriented pyrolytic graphite).

Particulate activating agents having particle sizes on the order of nanometers are preferred, for example particulate matter having particle size of less than about 100 nm, preferably less than about 50 nm, more preferably less than about 25 nm, and most preferably from about 2 to about 20 nm. The size of the particles is not critical except to the extent that where excellent edge resolution of a structure deposited in a reaction involving the particle is desired, the upper limit in size of the particle is reduced.

Especially preferred as activating agents are colloidal activating agents. As used herein, colloidal activating agent is meant to define particulate matter capable of being involved in a desired chemical reaction, such as a catalytic reaction and including plating of metal at surfaces, that is carried or surrounded by molecules that prevent agglomeration of the individual particles and that render the particulate soluble in, or at least able to be carried in, an organic or aqueous liquid. Suitable colloid-forming species and colloids are described in European patent publication no. 672765 by Reetz et al. The activating agent can comprise one or more metals of groups Ib, IIb, III, IV, V, VI, VIIb, VIII, lanthanides, and/or actinides of the periodic table prepared by cathodic reduction in the presence of a stabilizer. One method of preparation of such colloids is reduction, optionally with a supporting electrolyte, in organic solvents or in solvent mixtures of organic solvents and/or water within a temperature range of between about -78°C and about 120°C to form metal colloidal solutions or redispersible metal colloid powders, optionally in the presence of inert substrates and/or soluble metal salts of the respective metals. These colloids are soluble or redispersible in a suitable fluid that facilitates their application to an applicator such as a stamp. The following articles, incorporated herein by reference, describe as well exemplary activating agents suitable for use in connection with the invention. Vargo, et al., "Adhesive Electroless Metallization of Fluoropolymeric Substrates" *Science,* 262, 1711-1712 (December 10, 1993); Bönnemann, et al., "Preparation and Catalytic Properties of NR₄⁺ Stabilized Palladium Colloids", *Applied Organometallic Chemistry* 8, 361-378 (1994); Reetz, et al., "Size-Selective Synthesis of Nanostructured Transition Metal Clusters" *J*. *Am. Chem. Soc.* 116, 7401-7402 (1994); Reetz, et al., "Visualization of Surfactants on Nanostructured Palladium Clusters by a Combination of STM and High-Resolution TEM", *Science,* 267, 367-369 (January 20, 1995); and Meldrum, et al.,"Formation of Thin Films of Platinum, Palladium, and Mixed Platinum Palladium Nonocrystallites by the Langmuir Monolayer Technique" *Chem. Mater*., 7, 111-116 (1995).

Electrochemical methods are described in EP 672765, referenced above, for synthesis of soluble metal colloids by operating in an inert organic, aprotic solvent, with surface-active colloid stabilizers being added as the supporting electrolyte which will both prevent plating of the metal and protect, or stabilize, small metal nuclei in the cluster stage. A metal sheet serves as the anode to be dissolved and a metal or glassy carbon electrode serves as the cathode. After dissolution at the anode, the released metal salts are reduced again at the cathode, with tetraalkylammonium salts serving as stabilizers. Standard organic solvents can be employed.

Suitable exemplary stabilizers, or carriers, for the colloids, and at the same time as the supporting electrolyte, are quaternary ammonium or phosphonium salts R¹R²R³R⁴N⁺X⁻ and R¹R²R³R⁴P⁺X⁻, respectively. A wide variety of combinations of R¹, R², R³ and R⁴ are possible. Examples include the symmetrical tetraalkylammonium salts with R¹ = R² = R³ = R⁴ = n-butyl or n-octyl, mixed tetraalkylammonium salts with R¹ = R² = R³ = methyl and R⁴ = cetyl, or chiral tetraalkylammonium salts having four different residues. Aryltrialkylammonium salts may also be used. Suitable counter ions include various anions, e.g. halogenides (Cl⁻, Br⁻, I⁻), hexafluorophosphate (PF₆⁻), carboxylates R'CO₂⁻ (R' = alkyl, aryl), or sulfonates R"SO₃⁻ (R" = alkyl, aryl). A similar variety of phosphonium salts may be used, including tetraarylphosphonium salts, such as tetraphenylphosphonium bromide. Preferably, tetrabutylammonium chloride, bromide or hexafluorophosphate, tetraoctylammonium bromide, or tributylhexadecylphosphonium bromide can be employed. As metals, any of those listed above, in particular transition metals such as Fe, Co, Ni, Pd, Pt, Ir, Rh, Cu, Ag, or Au, are suitable. Suitable solvents are aprotic organic solvents, such as tetrahydrofuran (THF), toluene, acetonitrile (ACN), or mixtures thereof. The temperature in the electrolytic cell may be in the range between -78°C and + 120°C, preferably 15-30°C or room temperature.

A preferred activating agent is a colloidal catalyst that promotes deposition, for example electroless deposition, of a metal at region 34 of substrate surface 28 to which the colloidal catalyst is applied. For example, where fluid precursor 36 includes a suspension of a colloidal palladium catalyst, the fluid can be evaporated or adsorbed as described above, resulting in deposition of catalyst at regions 34 of substrate surface 28. Subsequently, an electroless copper plating bath can be introduced into channels 32 and deposition of copper allowed to take place at regions 34 of surface 28. Alternatively, micromold 20 can be removed from surface 28 and the entire surface 28 exposed to an electroless copper plating bath. Copper will plate only at those regions 34 of substrate surface 28 to which colloidal palladium catalyst had been applied. Electrochemical metal plating can be carried out as well. The chemically active agent of the invention can be any agent that can find use in chemical reaction, attraction, or other interaction proximate a substrate surface. Those of ordinary skill in the art will recognize a variety of agents that can be used in accordance with the invention, including, but not limited to solutions or suspensions of a very small species such as catalytic colloids, monomers, dissolved or suspended salts or ceramics or their precursors or other species.

According to yet another alternative a suspension of particulate species in a fluid carrier 36 can be introduced into channels 32, followed by removal of the fluid carrier via dissipation, as discussed. The particulate species can be organic, inorganic, or polymeric material as described above, for example finely-ground polymeric, ceramic, or crystalline material, or can be in the form of microspheres. The application of microspheres in a predetermined pattern to a substrate surface can serve a variety of purposes that will be apparent to those of ordinary skill in the art upon reading the present disclosure, in light of the state of the art as set forth in several publications. An article by Lenzmann, et al., entitled "Thin-Film Micropatterning Using Polymer Microspheres", *Chem. Mater*., 6, 156-159 (1994) describes formation of densely-packed monolayers of monodisperse polystyrene microspheres deposited on a glass substrate. The spheres serve as a mask for zinc sulfide deposition on the substrate as a thin film by thermal evaporation in vacuum. The mask (microspheres) are removed from the substrate surface after evaporative deposition leaving behind a surface with zinc sulfide features located in the interstitial spaces of the densely-packed spheres. For 2-micron diameter spheres, the lattice spacing of the resulting pattern is approximately 900 nanometers with individual trigonal pyramidal peaks. According to the present invention, a particular concentration of polymeric microspheres in a fluid carrier can be selected without undue experimentation that, when introduced into channels 32, followed by evaporation of the fluid carrier, would result in a monolayer of microspheres selectively patterned at regions 34 of substrate surface 28. Removal of micromold 20, followed by chemical vapor deposition, results in a well-ordered pattern of isolated, nano-scale regions of deposited material within the confines of region 34 of substrate surface 28.

An article by Dushkin, et al. entitled "Colored Multilayers From Transparent Submicrometer Spheres", *Langmuir*, 9, 3695-3701 (1993) discusses optical phenomena associated with polymeric beads at surfaces. Ordered multilayers are formed by evaporating the water carrier from polystyrene latex suspensions of diameter smaller than the wavelength of visible light. The spheres exhibit color when illuminated with polychromatic light. Accordingly, arrangement of a pattern of microspheres at regions 34 of substrate surface 28 in accordance with the invention can result in various radiative and colorimetric phenomena. An article by Hayashi, et al. entitled "Imaging by Polystyrene Latex Particles", *Journal of Colloid and Interface Science,* 144, 2, 538-547 (July, 1991), describes microarrays of identical images produced by polydispersed polystyrene particles at a surface. Microparticles and microbeads, especially polymeric particles and beads such as latex or polystyrene beads, find use in the field of biochemistry as solid supports for biochemical interaction. For example, a chemically or biochemically active agent can be coupled to a microbead or microparticle and optionally used in turn to immobilize a second agent that reacts with the immobilized agent, thereby immobilizing the second agent at a region at which the microbead is immobilized. That is, microbeads carrying a particular agent can be immobilized at a surface in a pattern using techniques of the invention and the patterned, immobilized beads can serve as locations for chemical reaction or biochemical interaction on the micro scale, for example as microreactors. Those of ordinary skill will recognize a variety of uses for patterned microparticles or microbeads carrying chemical or biochemical agents such as, for example, biochemical assays.

The pattern of parallel indentations 24 formed in surface 22 of micromold 20 is for illustrative purposes only. Any pattern, for example a pattern defined by a single indentation or many indentations, one or more of the indentations defining a non-linear pathway of uniform or non-uniform depth is intended to fall within the scope of the invention. Various patterns are illustrated in subsequent figures. The indentation pattern can be of a variety of dimensions and, according to one aspect of the invention, includes a region having a lateral dimension of less than 1 millimeter. "Lateral dimension" is meant to define a dimension parallel to application surface 22. According to preferred embodiments, the indentation pattern includes a portion having a lateral dimension of less than about 500 microns or less than about 100 microns, more preferably less than about 50, 20, or 10 microns, and more preferably still less than about 5 microns. According to a particularly preferred embodiment, an indentation pattern having a portion including a lateral dimension on the order of 1 micron is provided. The dimension of the indentations can be altered, as described in co-pending, commonly-owned U.S. Patent Application serial no. 08/397,635, filed March 1, 1995 by Whitesides, et al., entitled "Microcontact Printing on Surfaces and Derivative Articles", and International Patent Application Publication No. WO 96/29629.
by deforming article 20.

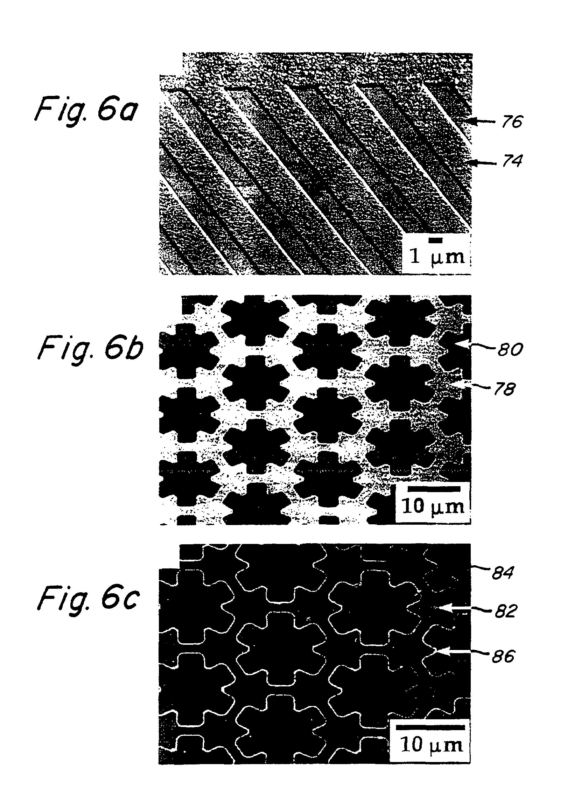

Where micromold 20 is placed adjacent a substrate surface and a fluid precursor fills channels 32, article or articles 38 resulting from the technique can have lateral dimensional features that correspond to the lateral dimensional features of indentations 32 of the micromold. According to another embodiment, the fluid precursor need not completely fill channels 32, and this is preferred according to embodiments in which the lateral dimension of article 38 formed from the fluid precursor is to be minimized. According to this embodiment, fluid precursor 36 is introduced into channels 32 in an amount small enough that the fluid precursor wets only the corners of the channels. When a fluid precursor, substrate, and micromold are selected such that the fluid precursor will wet the micromold efficiently via capillary action, when a small amount of fluid precursor is supplied to the mold channel or channels, the precursor will selectively wet portions of the channels having an interior angle relatively low relative to the rest of the channel (such as corners 40 defined by the abutment of contact surface 26 against substrate surface 28 at the edge of region 34 of the substrate surface). When the fluid precursor wets the corners selectively and the fluid is hardened, evaporated, or adsorbed, a resulting structure can define a pattern having a dimension smaller than that of the lateral dimension of indentation 24. According to this embodiment the lateral dimension of structure 38, at its narrowest, is narrower than the narrowest lateral dimension of channel 24 of the micromold, and can have a height significantly less than the height of the channel. The lateral dimension of article 38 according to this embodiment can be on the order of less than or equal to about 100 microns or 50 microns, or preferably less than about 20 or 10 microns, more preferably less than about 5 microns or 1 micron, and according to a particularly preferred embodiment less than approximately 0.2 micron. According to this aspect, any of the species described herein that can be patterned proximate a substrate surface can be patterned so as to have lateral dimensions as described above. This aspect of the invention is illustrated in Fig. 6c, and discussed below.

In an alternate technique, any of the species described herein that can be used to form patterned articles and the like on a substrate surface (such as fluid precursor 36) can be made to coat substrate surface 28, and then article 20 can be pressed against substrate surface 28 to displace precursor 36 at regions in register with contact surface 26. Precursor 36 will be formed in channels 32 as illustrated in Fig. 1, and procedures described above carried out.

Any suitable material can define substrate 30 of the invention. Substrate surface 28 can be of the same material as the bulk material of substrate 30, or a different material. Substrates exposing a variety of functional surfaces such as hydrophobic, hydrophilic, and biologically compatible or non-compatible surfaces are known, and are suitable for use with the invention. Substrates that are somewhat fluid are known as well, and are acceptable for use in the invention to the extent that a useful pattern can be formed thereupon. Article 20 similarly can be formed of essentially any material. For example, ceramic, polymeric, elastomeric, and other materials can be used. According to a preferred embodiment, substrate surface 28 and/or contact surface 26 of article 20 is an elastomer or other conformable material. Preferably, contact surface 26 and more preferably, for ease of fabrication, the entire article 20, is formed of an elastomer. When an elastomer defines substrate surface 28 or contact surface 26, or preferably micromold 20, an optimal seal is created between contact surface 26 and portions of substrate surface 28 adjacent and contiguous with portions 34 that with indentations 24 define channels 32. This results in optimal confinement of fluid precursor 36 to channels 32. Pressure can be applied to micromold 20 against substrate 30 during micromolding, but according to embodiments in which an elastomer is used as described, pressure need not be applied as the elastomer conforms well to the surface against which it mates thus sealing channels 32. The micromold 20 can be fabricated of an elastomer in a manner analogous to the fabrication of a stamp from an elastomer as described in co-pending, commonly-owned U.S. Patent No. 5,512,131, issued April 30, 1996 by Kumar, et al, entitled "*Formation of Microstamped Patterns on Surfaces and Derivative Articles",* and Internation Patent Application Publication No. WO 96/29629.

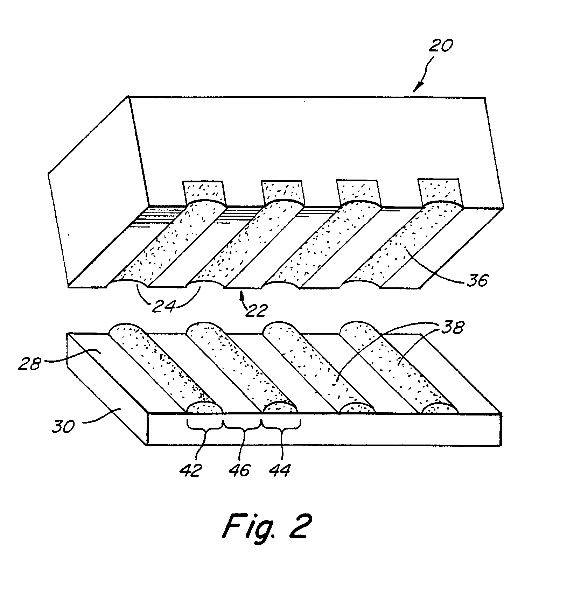

Fig. 2 illustrates another alternative in which, rather than applying article 20 to substrate surface 28 followed by introduction of fluid precursor 36 into channels 32 so defined, article 20 is used as an applicator to transfer a chemically or biochemically active agent (optionally in a fluid carrier), fluid precursor of an article such as microparticles or microbeads in suspension, catalytic colloid, prepolymer fluid, or the like to substrate surface 28. In Fig. 2 and subsequent figures, components common to the various figures are given common numerical designation. In Fig. 2, fluid precursor 36 is first applied to indentations 24 of micromold 20, and then application surface 22 is brought into proximity of substrate surface 28 to allow fluid precursor 36 to be transferred to substrate surface 28. The fluid precursor can be applied to the indentations by bringing the indentations into contact with the fluid precursor and allowing capillary action to cause the indentations to be filled, or the precursor can be applied via micropipetting or the like to the indentations. In this way, separate fluid species can be applied to separate indentations if desired. In can be advantageous, with these techniques, to select a material exposed by the contoured application surface and the fluid species applied thereto such that the fluid species rapidly is positioned within the indentations, rather than spreading over the entire surface. Those of skill in the art can carry out such selection, using contact angle measurements or the like.

Where fluid precursor 36 protrudes from indentations 24 prior to transfer, application surface 22 need not contact substrate surface 28 for transfer to take place. Typically, however, application surface 22 will be brought into contact with substrate surface 28 to transfer a pattern of the fluid precursor 38 to regions proximate the substrate surface in a pattern corresponding to the indentation pattern 24. As illustrated, some fluid precursor remains in indentations 24, and the fluid precursor transferred to substrate surface 28 has been converted into hardened article 38. However, according to several embodiments discussed above, the fluid precursor will not result in a hardened article, but will serve to transfer a biochemical agent or chemical agent to a surface. According to the embodiment illustrated in Fig. 2, the chemical or biochemical agent, prepolymer, fluid carrier containing a suspension of particulate matter, microbeads, or the like serves to transfer essentially instantaneously the desired species to the surface. As with all embodiments of the invention, the pattern of species so transferred can include a single indentation that is of any shape including a non-linear or linear pathway, a plurality of linear indentations as illustrated in Fig. 2, or a plurality of indentations of any shape, one or more indentations having dimensions as described above. Where a plurality of indentations are formed in application surface 22, each indentation can be made to carry a different chemical or biological agent or precursor. According to that embodiment, when application surface 22 of the micromold is brought into contact with substrate surface 28, distinct first and second species such as distinct first and second chemically or biochemically active agents, precursors, particulate species, or the like can be transferred essentially instantaneously to distinct first and second regions 42 and 44 proximate the substrate surface, in a pattern corresponding to the indentation pattern, and separated from each other by intervening region 46 of the substrate surface that remains free of the agent or precursor.

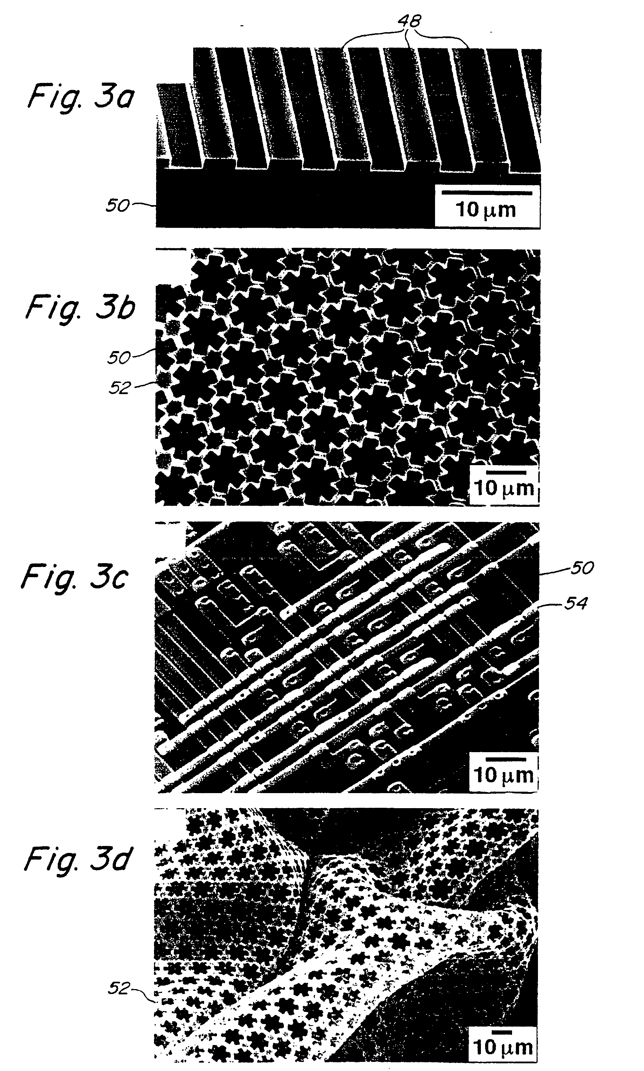

Figs. 3a-d are photocopies of SEM images of polymeric structures formed on substrates according to the method described above and illustrated in Fig. 1, in which a fluid polymeric precursor was allowed to fill channels formed by indentations in micromold 20 and regions of the substrate. Fig. 3a shows polyurethane articles 48 formed on Si/SiO₂ substrate 50 by capillary filling of a micromold having a surface with indentations placed adjacent substrate 50. The indentations correspond to the pattern of articles 48. A liquid polyurethane prepolymer was placed adjacent openings of channels formed between the micromold and the substrate surface and filled the channels via capillary action. The micromold was made of polydimethylsiloxane (PDMS). Fig. 3b is a top view of a polyurethane article 52 having a complex, interconnected pattern formed on Si/SiO₂ substrate 50. A PDMS micromold having an indentation pattern corresponding to the pattern of article 52 was used, and a liquid polyurethane prepolymer was allowed to fill the mold channels via capillary action. Fig. 3c shows a quasi three-dimensional array of microstructures interconnected through channels. Again, a polyurethane liquid prepolymer was allowed to fill channels formed by a micromold having an indentation pattern corresponding to the pattern of polyurethane article 54. Polyurethane article 54 is formed on a Si/SiO₂ substrate 50. Fig. 3d shows a free-standing patterned polyurethane article 52 formed by removal of the article from the substrate (Fig. 3b).

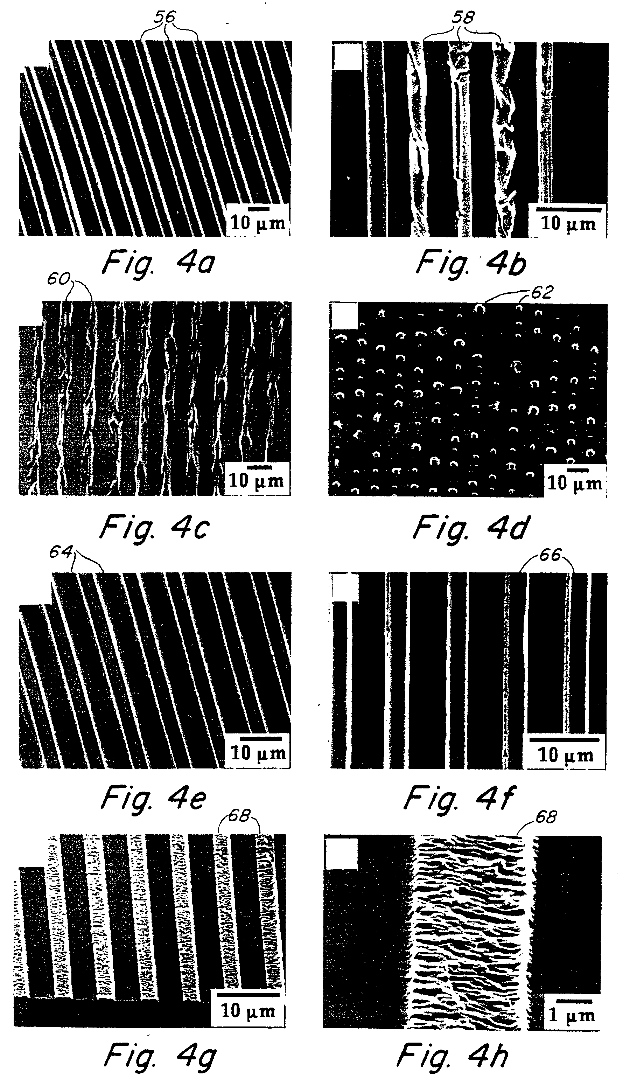

Figs. 4a-h illustrate structures formed on substrates using the micromolding technique illustrated in Fig. 1 in which liquid precursor 36 is a precursor of inorganic materials. Photocopies of SEM images are shown. In Fig. 4a, KH₂PO₄ structures precipitated from aqueous solution on Si/SiO₂ are shown. Fig. 4b shows KH₂PO₄ structures as well, crystallized more rapidly. Fig. 4c shows Cu(NO₃)₂ on the same substrate crystallized from aqueous solution. Fig. 4d shows structures formed of the same material on the same substrate, but crystallized from a much more dilute solution. Fig. 4d illustrates the derivatization in a pattern that is formed within the boundaries of a region of the substrate surface corresponding to the indentation pattern of the micromold, but that does not fill that region. A series of isolated regions of product on the order of 4 microns in lateral dimension are shown. Fig. 4e shows CuSO₄ structure on glass. Fig. 4f shows K₃Fe(CN)₆ structures on Si/SiO₂. Fig. 4g shows a fractured view of amaranth on glass. The structures are approximately 0.4 micron in height. Fig. 4h is a section of Fig. 4g at higher magnification.

Ceramic structures formed in accordance with the invention can find use, for example, as mechanical ceramics such as abrasion tools. Current methodologies involve, typically, chemical vapor deposition to form ceramic patterns having small dimensions for such uses.

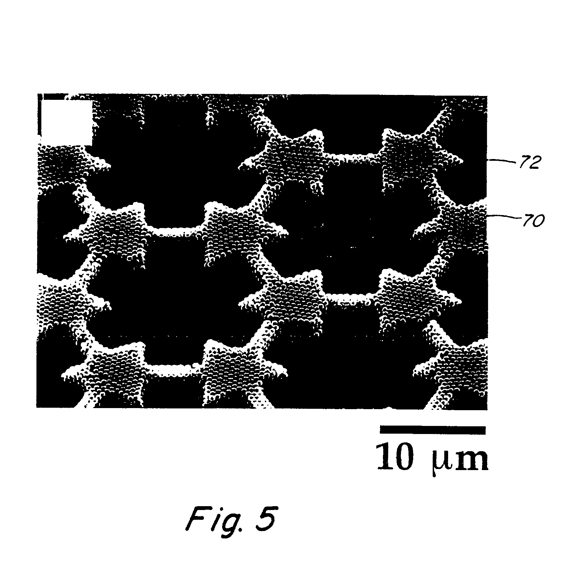

Fig. 5 is a photocopy of an electron micrograph showing a packed, ordered array of polystyrene microspheres 70 on a Si/SiO₂ substrate 72. The ordered array of microspheres was formed by allowing a latex solution containing polystyrene microspheres to fill, via capillary action, channels formed between a micromold and the substrate surface in a pattern corresponding to the pattern of microbeads shown. The PDMS micromold was removed following crystallization of the microspheres via dissipation of the fluid carrier.

Figs. 6a-c are photocopies of SEM images of copper structures formed via electroless deposition on Si/SiO₂ substrates. For the structure in Fig. 6a, a gold surface was provided. A PDMS micromold having an indentation pattern corresponding to the pattern of copper structures illustrated was placed adjacent the gold substrate (as illustrated schematically in Fig. 1) and the channels 32 were filled with a plating bath for electroless deposition of copper, defining a fluid precursor of copper according to one aspect, and a chemically active agent according to another aspect. The copper electroless plating solution was allowed to remain in contact with the surface for a period of time sufficient to plate copper structures 74 in a pattern corresponding to the indentation pattern of the micromold, while portions of gold surface 76 corresponding to contact surface 26 of the micromold remained free of copper deposition. For the structure illustrated in Figs. 6b-c, a PDMS micromold having an indentation pattern corresponding to the pattern of copper structures illustrated was placed adjacent the substrate and the channels 32 were filled with a precursor solution 36 containing catalytic colloids. The solvent in which the catalytic colloids dissipated, resulting in formation of the catalytic colloids as a chemically active agent formed on regions of the substrate surface corresponding to the indentation pattern of the micromold. The micromold was removed, and the surface exposed to an electroless copper plating bath. Specifically, in Fig. 6b, copper structures 78 were formed on a Si/SiO₂ substrate 80 coated with a self-assembled monolayer of siloxane on the Si/SiO₂ substrate. CH₃CH₂O)₃Si(CH₂)₃NH₂ defined the self-assembled monolayer. A micromold having an indentation pattern corresponding to the ultimate copper pattern 78 was placed on the self-assembled monolayer-derivatized silicon substrate. A DMF solution containing palladium colloids as a fluid precursor 36 was allowed to fill the channels. Dissipation of DMF resulted in the chemically active agent (specifically, palladium colloid) forming structures in a pattern corresponding to pattern 78. The substrate was exposed to an electroless copper plating bath to plate copper at patterned region 78. Fig. 6c illustrates an aspect of the invention in which articles of very small lateral dimension can be formed by allowing a small volume of fluid precursor 36 to enter channels 32 defined by the micromold indentations and the substrate surface. The substrate was prepared as described in connection with Fig. 6b. A region 82 of the substrate surface corresponds to the indentation pattern of the micromold. The fluid precursor 36 wetted only the corners defined between the substrate surface 84 and the micromold channels thus, when the fluid carrier dissipated, the catalytic colloid was solidified only in those portions of the indentation pattern that were wetted, namely, the comers. When the surface was exposed to an electroless copper plating bath, copper was plated at the regions 86 to which the catalytic colloid had been deposited. A copper pattern of very small lateral dimension resulted.

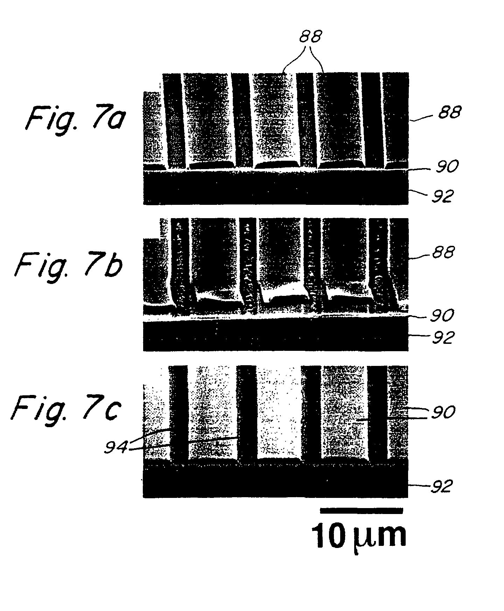

Figs. 7a-c illustrate the application of a patterned structure to a surface from a fluid precursor using micromolding as illustrated in Fig. 1, followed by use of the structure as a resist in a chemical etch. A polymeric structure 88 (polyurethane) was formed from a fluid prepolymer in a pattern corresponding to an indentation pattern on a 200 nm, thermally grown oxide layer 90 of a silicon substrate 92 (Fig. 7a). Following exposure of the substrate to a solution (aqueous HF/NH₄F for about 2 minutes) that etches silicon dioxide, but to which the polymeric structure 88 was resistant, the silicon dioxide layer was removed at regions of the substrate intervening the regions covered by the patterned structure 88, that is, regions that had been contacted by contact surface 26 of the micromold (Fig. 7b). Subsequently, the substrate surface was exposed to a solution (400 ml H₂O, 92g KOH, 132 ml 2-propanol for about 15 minutes at 65 °C) that etches silicon, but to which silicon dioxide is resistant. Fig. 7c shows resultant channels 94 anisotropically etched in the silicon substrate between patterned regions of silicon dioxide 90 that correspond to the pattern of polymeric structure 88 formed on the substrate surface via the micromolding technique.

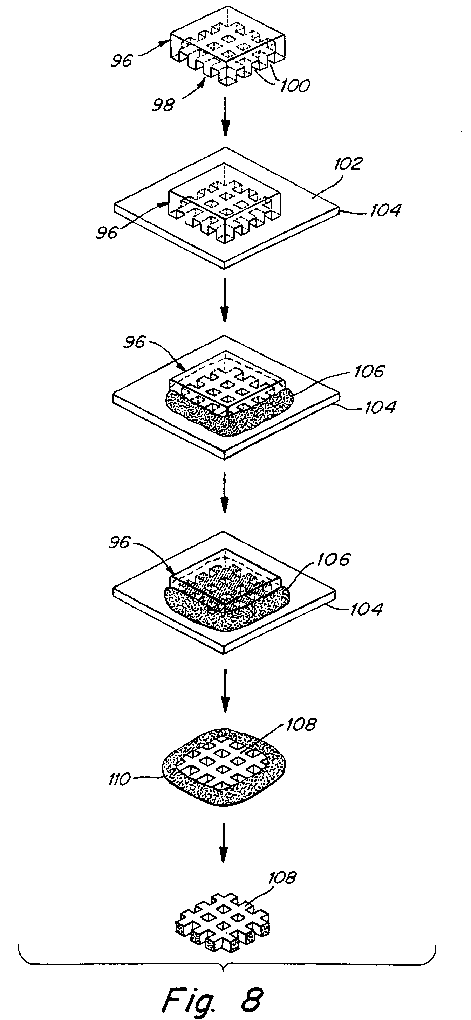

Fig. 8 illustrates schematically a technique for forming a mask, for use in lithography or the like, via the micromolding technique of the invention. A micromold 96 having a molding surface 98 including a plurality of indentations 100 in a grid-like pattern is applied to a surface 102 of a substrate 104. A fluid polymeric precursor 106 is placed adjacent openings of channels formed between the substrate surface and the indentations of the micromold, and allowed to flow, via capillary action, into the channels. Where a PDMS micromold was used, the polymeric precursor could be placed so as to cover all channel openings, and flowed into and made to fill the channels completely. Gas escaped presumably via diffusion through the micromold. Once the polymeric precursor was hardened, via thermal or photolytic polymerization or the like, the micromold was removed. The substrate then was separated from the resultant patterned article 108. The patterned article had a "frame" 110 completely surrounding it which could be used for ease of manipulation. The frame could be removed as well, to form the article 108 in a pattern corresponding to the indentation pattern of the micromold free of the frame.

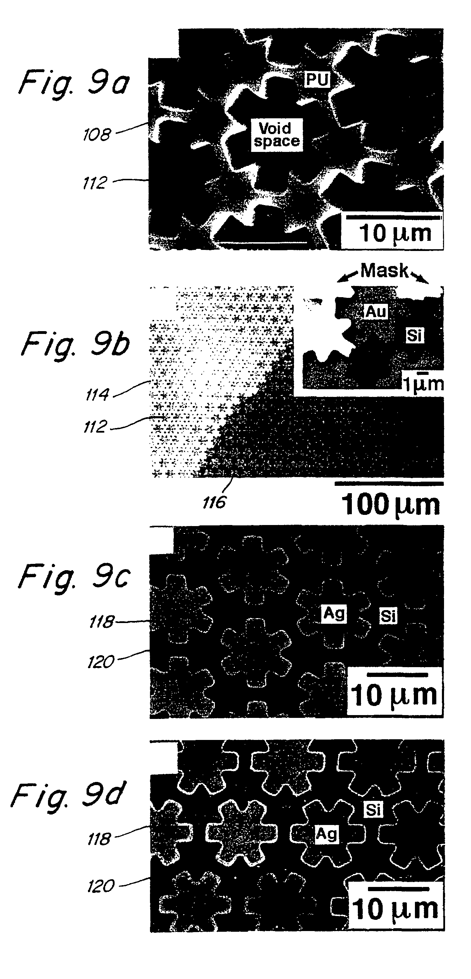

The article 108 could be used as a mask, for example as illustrated in Figs. 9a-d, which are photocopies of SEM images. Fig. 9a shows a polyurethane mask 108 formed as illustrated in Fig. 8, and following formation placed on a Si/SiO₂ substrate 112. Fig. 9b shows the mask 108 on the substrate 112 following evaporation of gold onto the substrate. A portion of the mask was removed and mask 114 and portions 112 of the substrate not covered by the mask are shown covered with gold. Portions 116 of the substrate that had been covered by mask 108 remain free of gold. Fig. 9c shows a surface having a pattern of isolated regions 118 of gold on a silicon substrate (regions 120 of the silicon substrate not covered by regions 118 of gold can be seen) formed as follows. A mask fabricated as described above was placed (with reference to Fig. 8) on a silicon substrate carrying a thin film of gold. A self-assembled monolayer-forming species (hexadecanethiol) was exposed to the surface and formed a self-assembled monolayer selectively at regions 118 not covered by the mask. The mask then was removed from regions 120, and the surface exposed to a solution that etched gold, but to which the self-assembled monolayer was resistant. The self-assembled monolayer then was removed, resulting in the regions 118 of gold that had been protected by the secondary, self-assembled monolayer resist, isolated by regions 120 of the silicon substrate. Fig. 9d shows a surface derivatized as described with respect to Fig. 9c. but the self-assembled monolayer was transferred to regions 118 of the surface by placing a flat PDMS article that had been coated with a self-assembled monolayer-forming species on top of the mask 108 for one minute.

Mask 108 also could be applied to nonplanar surfaces followed by plating, etching, or the like. It can be advantageous, when transferring mask 108 to a surface having very fine features, such as a surface etched as illustrated in Fig. 7c, to transfer mask 108 to such a surface by floating it in a fluid that is supported by the surface and allowing the fluid to dissipate or run off.

As described in co-pending, commonly-owned U.S. Patent Application Serial Number 08/397,635 of Whitesides, et al., referenced above, etching or plating at a surface can be made to take place selectively at predetermined regions, and this technique can be exploited using the techniques of the present invention as described herein. Additionally, where a self-assembled monolayer is patterned, a "protecting species" that is resistant to (for example, incompatible with) a chemical etch can be placed on top of a self-assembled monolayer, followed by etching at regions not covered by the self-assembled monolayer, as described in application Serial No. 08/397,635. Of course, a self-assembled monolayer can be incompatible with an etch and etching can take place without the use of a protecting species. The protecting species, according to this embodiment, is compatible with the self-assembled monolayer. According to another embodiment, a protecting species is less compatible with the self-assembled monolayer than with the substrate surface that is exposed at regions intervening the self-assembled monolayer. According to this embodiment, after patterning of a self-assembled monolayer a protecting species is exposed to the surface and when the surface is exposed to an etchant, the surface is etched at regions that had been covered by the self-assembled monolayer.

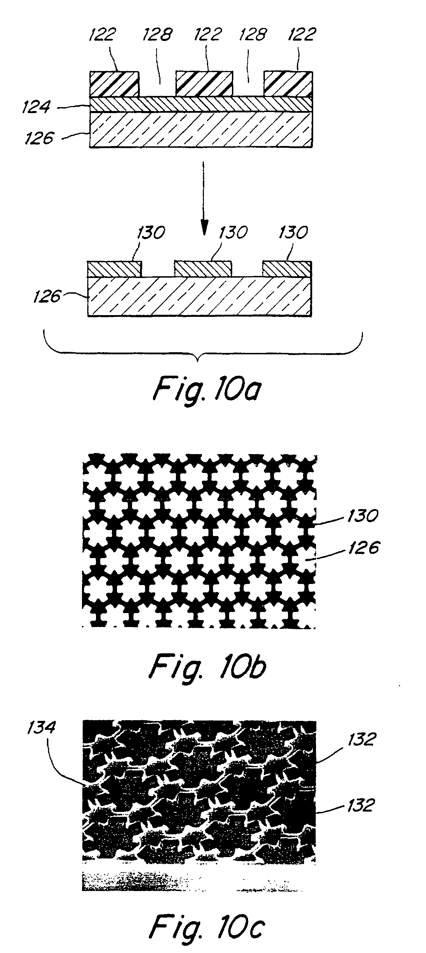

Figs. 10a-c illustrate formation of a mask 122 on a thin layer 124 of chromium on a glass substrate 126 using the micromolding procedure as illustrated in Fig.1, followed by etching of chromium at regions 128 not covered by the mask. In Fig. 10a, the molding technique described above is used to form a pattern of polyurethane article 122 on chromium 124 leaving region 128 of chromium uncovered. The surface was exposed to an etchant (400 ml H₂O, 24 ml of 63% HNO₃, 62g NH₄NO₃.Ce(NO₃)₃ for about 1 minute) that removes chromium but to which the polymeric article 122 is resistant. The result was a glass substrate 126 having thereon a patterned mask 130 defined by chromium protected from the etch by mask 122 in the pattern corresponding to mask 122 (and the pattern corresponding to the indentation pattern of the micromold). Fig. 10b is an optical micrograph of the chrome mask 130, top view. The chrome mask 130 was removed from the substrate 126 and placed on a photoresist article. Fig. 10c is a photocopy of an SEM image of a pattern that was generated in the photoresist film at regions 132 not protected by the mask. Raised portions 134, in a pattern corresponding to the pattern of the chromium mask, and corresponding to the original indentation pattern of the micromold from which the mask 122 was formed, were not ablated in the photolithography process.

According to another embodiment a substrate surface such as a silicon wafer can be spin-coated with photoresist. A micromold can be placed adjacent to a photoresist and channels defined thereby filled with a solvent that dissolves photoresist but not the micromold. A pattern of the silicon wafer not covered by photoresist, the pattern corresponding to the indentation pattern of the micromold, is thereby produced. Further processing familiar to those of ordinary skill in the art can be carried out.

Fig. 11 illustrates schematically an applicator 136 that can be used for applying any of the above-described chemically or biochemically active agents, polymeric precursors, fluid precursors of solid structures, fluid carriers of particulate matter, and the like to a substrate surface. Applicator 36 includes a plurality of isolated indentations 138 separated from each other by intervening regions of a surface 140 in which the indentations are formed. As illustrated, two of the indentations contain fluid carrier 142 and fluid carrier 144, respectively. The fluid carriers 142 and 144 can be the same or different. A substrate 146 is shown that, for purposes of illustration, includes a self-assembled monolayer 148 formed thereon which can serve as an adhesion promoter. Fluid carriers 142 and 144 are transferred to isolated regions proximate the surface of substrate 146, in particular, regions of the exposed self-assembled monolayer 148 on the surface of substrate 146. The transfer typically takes place by bringing the surface 140 of the applicator into contact with the self-assembled monolayer 148 but, if the fluid carriers 142 and 144 protrude from the indentations, the surface 140 need only be placed in close proximity to the self-assembled monolayer 148.

Fluid carriers 142 and 144 can be any of the species described above and, according to a particularly useful embodiment, carry or define a chemically or biochemically active agent that can be used in a subsequent assay or the like. For example, a self-assembled monolayer 148 can be a monolayer of a species X-R-Ch as described in co-pending commonly-owned application serial no. 08/312,388, by Bamdad, et al., entitled "Molecular Recognition at Surfaces Derivatized with Self-Assembled Monolayers".
These species include have the general formula as above where X represents a functional group that adheres to a gold surface, R represents a spacer moiety that promotes formation of a self-assembled monolayer of a plurality of the molecules, and Ch represents a bidentate, tridentate, or quadradentate chelating agent that coordinates a metal ion. The chelating agent includes a chelating moiety and a non-chelating linker moiety, such that it can be covalently linked via its linker moiety to the spacer moiety while allowing the chelating moiety to coordinate a metal ion. According to a preferred aspect of the invention a metal ion is coordinated to the chelating agent, and a binding partner of a target molecule is coordinated to the metal ion. This arrangement is facilitated by selecting the chelating agent in conjunction with the metal ion such that the chelating agent coordinates the metal ion without completely filling the ion's coordination sites, allowing the binding partner to coordinate the metal ion via coordination sites not filled by the chelating agent. A non-limiting exemplary list of suitable chelating agents includes nitrilotriacetic acid, 2,2'-bis(salicylideneamino)-6,6'-demethldiphenyl, and 1,8-bis(a-pyridyl)-3,6-dithiaoctane. The binding partner can be a biological species that includes a polyamino acid tag, such as a tag made up of at least two histidine residues, that coordinates the metal ion. In this context the term "adhere" means to chemisorb in the manner in which, for example, alkyl thiols chemisorb to gold.

In this case the fluid carriers 142 and 144 can be carriers of a nickel ion, resulting in a surface suitable for capture of a biological binding partner carrying a polyamino acid tag selectively at regions to which carriers 142 and 144 had been applied. According to this embodiment, immediately following application of carriers 142 and 144 to the substrate surface, it can be advantageous to expose the surface of substrate 146 to a chelating agent in solution to remove excess nickel ion from the surface. In this way, stray uncoordinated nickel ion does not coordinate to the self-assembled monolayer 148 at regions outside of those regions to which carrier 142 and 144 had been applied. The latter-applied chelating agent preferably less-strongly coordinates nickel ion than the chelating agent immobilized at the surface. Following this application step, a plurality of isolated regions of self-assembled monolayer 148 include nickel ion. Accordingly, when the surface is exposed to a polyamino acid-tagged biochemically active agent, the biochemically active agent will attach selectively at those regions to which nickel ion had been applied.

According to another alternative, self-assembled monolayer 148 can be a species X-R-Ch-M as described in the above-referenced co-pending application serial no. 08/312,388, and the species 142 and 144 can be polyamino acid-tagged biological binding partners, optionally contained in a fluid carrier, that are attached to the surface selectively at those regions corresponding to the indentation pattern of the applicator. According to this embodiment the separate, isolated regions can include separate, distinct biochemically active agents. This can be accomplished, for example, by placing applicator 136 in register with a plurality of reservoirs of distinct (different) biochemically active agents to position distinct biochemically active agents in the respective indentations of the applicator, then placing the applicator adjacent the surface of substrate 146 to transfer distinct biochemically active agents to distinct, isolated regions of the surface. The procedure can be repeated using fresh substrate surfaces for each step, thus surfaces carrying distinct regions of distinct biochemically active agents can be mass produced. In addition to the species described above, cells can be immobilized at a substrate surface in this manner as well. Register between the applicator and the substrate surface can be controlled via mechanical, electronic, magnetic. and/or optical apparatus.

According to another alternative, species 142 and 144 or species carried by fluid carriers 142 and 144 can be transferred to a surface carrying a self-assembled monolayer other than the monolayer of X-R-Ch-M as described above. For example, a self-assembled monolayer exposing a hydrophobic functionality such as an alkane functionality can be formed on a surface (e.g., hexadecanethiol on gold) and a biochemically or chemically active agent that adheres to a hydrophobic surface can then be applied to the surface in discrete regions or in a pattern as described above. Where the biochemically active agent is a cell or cells, it may be advantageous to coat the hydrophobic surface with a cytophilic species such as laminin. Reference can be made to U.S. Patent Application Serial No. 08/131,838 of Sighvi, et al. referenced above, in connection with the immobilization of cells at surfaces.

Immobilization of cells and other biochemically active species can be carried out without a self-assembled monolayer as well. For example, a hydrophobic surface coated with laminin, and free of self-assembled monolayer, can serve as a substrate for immobilization of a pattern of cells in accordance with the invention.

According to this and other alternatives, the substrate surface can carry chelating agent immobilized via other than a self-assembled monolayer. For example, chelating agents coupled to dextran at a surface, as is known, can be employed. Although a self-assembled monolayer 148 is illustrated on the surface of substrate 146, a self-assembled monolayer is not needed according to all embodiments. For example, substrate 146 can be adhesive to a species transferred to it from applicator 136, for example a biochemically or chemically active agent and fluid carriers 142 or 144, or the like. Additionally, the applicator can be placed in contact with the substrate surface and allowed to remain in place while any species present in the fluid precursor is allowed to harden, the fluid carrier is allowed to dissipate, or the like.

In accordance with alternatives, such as those illustrated in Figs. 1, 2, and 11, the species formed proximate the substrate surface in a pattern corresponding to the indentation pattern of the article itself can be a self-assembled monolayer. Suitable self-assembled monolayer-forming species are described in U.S. Patent Application Serial Nos.08/131,841 of Kumar, et al., referenced above. Self assembled monolayers formed of species X-R-Ch, as described above, with or without metal ion and/or biological species coordinated thereto, can be used, as well as other self-assembled monolayer-forming species disclosed in application serial no. 08/312,388, by Bamdad, et al., referenced above.

Fig. 12 illustrates schematically a process for applying a species from indentations in an applicator to a non-planar surface. An applicator 136 (shown in cross section) includes a plurality of indentations 138, each filled with a species 150. Each of the indentations can be filled with the same fluid or different fluids. Species 150 can be any of the above-described fluid precursors, chemically or biochemically active agents, or the like. An article 152 having a surface 154 is placed adjacent the application surface of applicator 136 and rolled against the applicator as described in commonly-owned, co-pending U.S. patent application serial no. 08/397,635 by Whitesides. et al., entitled "Microcontact Printing on Surfaces and Derivative Articles", and Internation Patent Application Publication No. WO 96/29629.

As the article 152 including nonplanar surface 154 is rolled against the applicator 136. species 150 is transferred to surface 154 in a pattern corresponding to the indentation pattern of the applicator. The indentation pattern can be any pattern as described above, for example individual, isolated regions or one or more continuous linear or non-linear indentations. The indentation or indentations can be of one or more depths. Application to nonplanar surfaces having various radii of curvature can be carried out according to the invention, for example, radii of curvature of less than about one centimeter, preferably less than about one millimeter, more preferably less than about 500 microns, more preferably less than about 100 microns, more preferably less than about 50 microns, and according to a particularly preferred embodiment printing can occur on substrates with radii of curvature on the order of about 25 microns or less.

Fig. 13 illustrates an article 155 created by forming, on a silicon dioxide surface 156 of a silicon substrate 158, a patterned structure 160, for example a polymeric structure formed from a prepolymeric fluid using a micromold as illustrated in Fig. 1. Subsequently, a second fluid precursor is positioned so as to cover the patterned structure 160 and allowed to solidify. According to the embodiment illustrated, a fluid precursor was placed atop the patterned structure 160 and a micromold having a complex pattern was placed atop the fluid precursor. The fluid precursor was hardened to form a structure 162 covering and encompassing the patterned structure 160 on the substrate surface. The second structure 162 included an exposed surface 164 having a pattern of indentations 165 complementary to the indentation pattern of the second micromold. The overall structure, when structure 160 differs in refractive index from structure 162, can serve as a waveguide, the second structure 162 serving as a cladding. The contoured surface 164 of cladding 162 is lossy. The pattern of surface 164 is not important to the waveguide function. Waveguides were fabricated from several classes of polymeric materials (epoxies, polyurethanes, and polyacrylates on Si/SiO₂ substrates. Waveguides clad with polymers having slightly lower refractive indices gave single-mode output in the visible and near infrared regions. A typical waveguide structure exhibiting single-mode behavior consisted of a trapezoidal waveguide (n_{guide} = 1.545) clad in a polymeric slab with n_{cladding} = 1.52. The waveguide was 0.7 centimeters long and the wavelength of light was 0.85 micron. Photocurable polymers are preferred.

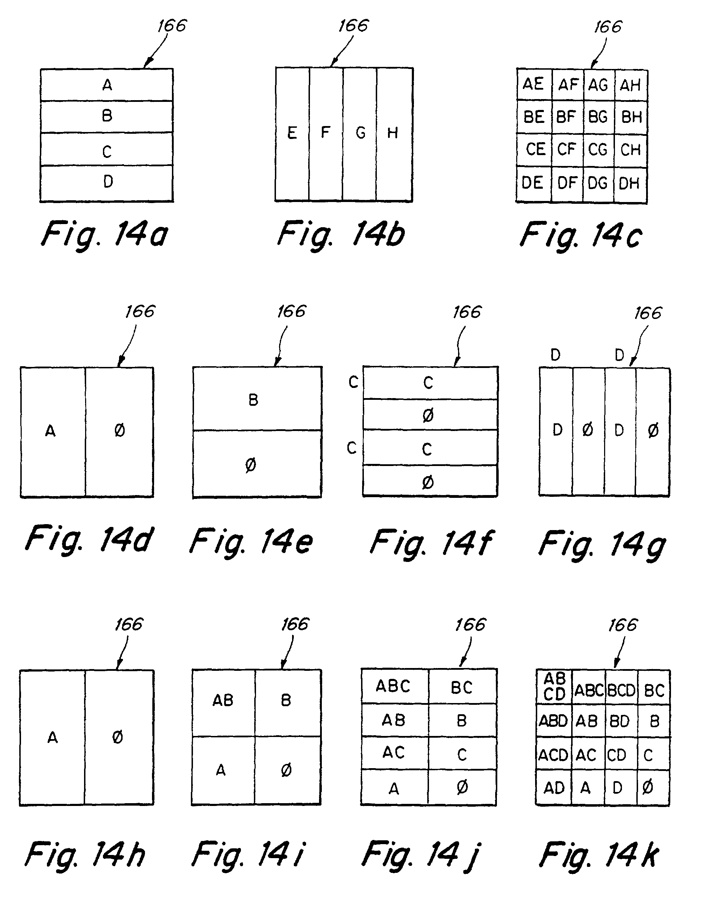

Referring now to Figs. 14a-k, a schematic illustration of a surface derivatized so as to include discrete regions of differing chemical functionality is shown. The article schematically illustrated finds particular use as a combinatorial library. An article by Jacobs, et al., entitled "Combinatorial Chemistry―Applications of Light-Directed Chemical Synthesis", *Trends in Biotechnology,* volume 12, 19-26 (January, 1994) describes a photolithographic process for forming a combinatorial library. Jacobs, et al. describe derivatizing a substrate with linker molecules that contain amines blocked by a photochemically cleavable protecting group. Specific sites on this synthesis surface are photo-deprotected by illumination through a photolithographic mask. Those regions exposed to light are deprotected, and may then be coupled to amino acids of interest using standard peptide-synthesis conditions. The process is repeated using new masks until an array of compounds of the desired length and composition are built up. The patterns of photolysis and order of addition of amino acids define the products and their locations on the solid support. The present invention can find application in combinatorial library synthesis with a minimum of expense and equipment. Those of ordinary skill in the art will recognize that any of a wide variety of chemically and biologically active agents can be used in accordance with the procedure discussed below and illustrated in Figs. 14a-k. The criteria for selection of such agents is similar to selection criteria for those agents described above that can be positioned on a substrate surface using a forming article or micromold 20. In a manner analogous to the procedure of Jacobs, et al., wet chemical protecting groups can be utilized in accordance with the present invention rather than photochemically cleavable protecting groups. Orthogonal-stripe methods and binary synthesis of combinatorial libraries in accordance with the invention, with reference to Figs. 14a-k, are described below in the prophetic example.

The following prophetic example involves the creation of a combinatorial library on a substrate surface using the micromold of the invention. With optional reference to Jacobs, et al., "Combinatorial Chemistry―Applications of Light-Directed Chemical Synthesis", *Trends in Biotechnology,* 12, 19-26 (January, 1994) and *Chemical & Engineering News,* 74, 7, 28-73 (Feb. 12, 1996), those of ordinary skill in the art can follow the teachings herein to form a combinatorial library inexpensively.

Reference will be made to Figs. 14a-k, which illustrate schematically top views of a substrate surface. Figs. 14a-c illustrate an "orthogonal-stripe" method. According to the technique, a plurality of micromolds are fabricated, each of which has a distinct channel pattern. Each micromold is fabricated so as to cover substrate surface 166, or at least enough of substrate surface 166 to define a channel or channels necessary for application of chemically or biochemically active agents to desired regions of the surface. For purposes of illustration, the description will assume use of a micromold that completely covers substrate surface 166, and includes indentations in register with certain portions of substrate surface 166. One micromold includes an indentation in register with a portion of the substrate surface designated "A" in Fig. 14a and includes a contact surface that contacts the remaining substrate surface at areas designated "B", "C", and "D". With reference to Fig. 14b, individual micromolds will be fabricated that include contact surfaces that cover all portions of the substrate except one of the portions "E", "F", "G", or "H". That is, each micromold forms a channel through which a chemically or biochemically active agent (reactant) can be delivered to the substrate surface at a portion in register with the channel, while remaining portions of the micromold block regions proximate the substrate surface from interaction with the particular chemically or biochemically active agent. Any combination of micromolds can be used to apply to the surface, in any combination, various chemically or biochemically active agents. For example, with reference to Fig. 14a, if a micromold having a channel in register with region "A" of the substrate surface is used to apply to the surface a chemically active agent "A" and then, with reference to Fig. 14b, a micromold is placed adjacent the substrate surface that has an indentation in register with region "E" and is used to apply to region "E" a chemically active agent "E", the substrate surface will include a region carrying chemically active agent "A" (the region designated "A" in Fig. 14a), a region carrying chemically active agent "E" (the region designated "E" in Fig. 14b), and at the region where the regions "A" and "E" intersect both chemically active agents will have been applied (upper left corner of the substrate surface as viewed in Fig. 14c). It can be seen that, if all combinations of micromolds and chemically active agents are employed, the result will be a grid on the substrate of each combinatorial permutation of the chemically active agents each confined to a separate region of the substrate surface (Fig. 14c). As is apparent to those of ordinary skill in the art, the order of application of active agent to the various regions of the substrate surface can be used to tailor the synthesis of the individual species at the various locations on the substrate surface.

With reference to Figs. 14d-k, a "binary" synthesis technique is described. In Fig. 14d, a first micromold having an indentation in register with region "A" and a contact surface in register with region "Ø" is used to apply to the substrate surface an active agent "A" selectively at region "A". Fig. 14b shows surface 166 including a region "B" in register with an indentation of a second micromold and a region "Ø" in register with a contact surface of the second micromold, via which an active agent "B" can be applied selectively to region "B" of the substrate surface. Fig. 14f shows surface 166 having portions "C" and "Ø" that are positionable in register with indentations and contact portions, respectively, of a third micromold to apply an active agent "C" to regions "C". In Fig. 14g the surface includes portions "D" and "Ø" that are positionable in register with indentations and contact portions, respectively, of a fourth micromold to transfer agent "D" selectively to regions "D". The binary technique is less labor intensive than the orthogonal-stripe method in that only four transfer or flow steps involving four micromolds are needed to create a grid of sixteen distinct chemically or biochemically functional regions on the substrate surface.

After application of the first micromold and formation of chemically or biochemically active agent "A" via the channel of the first micromold, agent "A" is applied to the left side of the substrate surface 166 and the right side of the substrate surface remains free of agent as illustrated in Fig. 14h. After application of agent "B" to the upper portion of substrate surface using the second micromold, four quadrants of the substrate surface carry agent "A" plus agent "B", agent "B", agent "A", and no agent, respectively, as illustrated in Fig. 14i. After application of agent "C" via the channels of the third micromold, eight regions of distinct chemical or biochemical functionality exist on the surface as illustrated in Fig. 14j. After application of agent "D" via the indentations of the fourth micromold, sixteen distinct chemically or biochemically active regions are formed as illustrated in Fig. 14k, namely "ABCD", "ABC", "BCD", "BC", "ABD", "AB", "BD", "B", "ACD", "AC", "CD", "C", "AD", "A", "D", and "Ø". The register between each micromold and the substrate surface can be controlled by pins in the substrate that engage each micromold, pins in each micromold that engage the substrate surface, an X-Y table that positions the substrate surface identically relative to each micromold, optical, magnetic, or electronic aligning apparatus, or other equivalent apparatus that can align each micromold with the substrate surface. Accurate register at the micron scale is achieved.

Those of ordinary skill in the art have the ability to select, without experimentation or with only routine experimentation, chemically or biochemically active agents that can be used to create a myriad of chemically active or biochemically active combinatorial libraries according to the technique of the invention. It can be useful to first coat substrate surface 166 with a common chemical linker functionality coupled to a chemical protecting group, apply a first micromold to the substrate surface, first deprotect at the region in register with the channel, then carry out a synthesis step at that region and reprotect, then remove the first micromold and apply a second, different micromold, again deprotect at the portion of the substrate surface in register with the channel of the second micromold, carry out a second synthesis step, and reprotect, etc. Libraries of peptides, synthetic molecules such as new drugs, naturally-occurring chemical and biochemical species, oligonucleotides and the like can be created. Indeed, any of the chemically or biochemically active agents, fluid precursors, prepolymeric fluids, or the like as described above that are transferable from a microapplicator or that can be applied, for example via capillary action, to a surface using a micromold as described above, can find use in the combinatorial arrangement described. Any combination of various agents can be used.

As an alternative to that described above, an article 20 as illustrated in Fig. 2, having a contoured surface 22 including a plurality of protrusions separated by intervening indentations 24 can be used as a stamp for forming a combinatorial library. Stamping as described in U.S. Patent Application Serial No. 08/131,841 (Kumar, et al.,) can be employed. The stamp includes a stamping surface defined by the outer surfaces of the protrusions. The process is described with reference to Figs. 14d-k. A surface 166 carries a protecting group, for example, a self-assembled monolayer exposing outwardly an azide functionality. A stamp having a surface including a protrusion in register with area A of surface 166 (Fig. 14d) is prepared by applying to the protrusion a deprotecting species such as a reducing agent for reduction of the azide to a deprotected, reactive amine. Application of the stamp to surface 166 deprotects the self-assembled monolayer at region A, but leaves the remainder of surface 166 (Ø) protected. Then, chemical reactivity at region A can take place, followed by reprotection of the entire surface. Then the stamp can be reoriented, or a second stamp chosen, so that region B is deprotected by contact with a stamping surface (protrusion) of a stamp. Chemical reaction then is carried out a region B, and the surface re-protected. With reference to Fig. 14f, a stamp having protrusions corresponding to regions C is used to deprotect at regions C, followed by chemical reaction at regions C and re-protection, and the process carried out similarly at regions D (Fig. 14g). According to a preferred embodiment, the stamping surface itself, without any auxiliary agent carried thereon, can deprotect at regions of surface 166 in register with the stamping surface. For example, a stamp having an acidic stamping surface such as a hydrogel loaded with a component of low pH can be used. For example, Dextran™ carrying polyphosphoric acid can be grafted to a surface of a rigid or elastomeric stamp and used to deprotect surface 166 at regions corresponding to the protrusions or stamping surface. Other protecting/deprotecting chemistries such as hydrolysis chemistry can be carried out.

According to another alternative, rather than building a combinatorial library through step-by-step synthesis of various species at various distinct regions proximate a substrate surface, distinct species can be synthesized and applied to the substrate surface after synthesis. A combination of these approaches can be used as well, involving synthesis of building blocks that are assembled according to the prophetic example.

## Claims

1. A method of creating a pattern of a species (38) at a first region (34) proximate a substrate surface (28), the pattern including a portion having a lateral dimension of less than 1 mm, while leaving a second region proximate the substrate surface (28), contiguous with the first region (34), free of the species, using an article (20) having a contoured surface (22) including at least one indentation (24) defining a pattern,
**characterized in that** the method involves creating the pattern of the species (38) at the first region (34) proximate the substrate surface (28) in a manner that corresponds to and is in register with the indentation pattern of the article (20).

2. A method as in claim 1, comprising:
forming at the first region (34) proximate the substrate surface (28), in a pattern corresponding to the indentation pattern, a species that is a fluid precursor of an inorganic species: and
allowing the fluid precursor to harden at the first region (34) of the substrate surface (28) as an inorganic species in a pattern corresponding to the indentation pattern.

3. A method as in claim 1, comprising:
forming at the first region (34) proximate the substrate surface (28), in a pattern corresponding to the indentation pattern, a suspension of particles in a fluid carrier: and
allowing the fluid carrier to dissipate thereby depositing the particles at the first region (34) of the substrate surface (28) in a pattern corresponding to the indentation pattern.

4. A method as in claim 3, wherein the particles are microbeads.

5. A method as in claim 1, comprising:
forming at the first region (34) proximate the substrate surface (28), in a pattern corresponding to the indentation pattern, an agent capable of promoting deposition of a metal.

6. A method as in claim 5, wherein the agent is formed at the substrate surface (28) in a fluid carrier, the method involving allowing the fluid carrier to dissipate thereby depositing the chemically active agent at the first region (34) of the substrate surface (28).

7. A method as in claim 1, comprising:
forming at the first region (34) proximate the substrate surface (28) in a pattern corresponding to the indentation pattern, a fluid precursor of the species by transferring the fluid precursor from the contoured surface of the article (20) to the first region (34) of the substrate surface (28) in the pattern; and
allowing the fluid precursor to harden at the first region (34) of the substrate surface (28), in a pattern corresponding to the indentation pattern.

8. A method as in claim 7, involving transferring the fluid precursor essentially instantaneously from the contoured surface of the article (20) to the first region (34) of the substrate surface (28).

9. A method as in claim 8, wherein the pattern includes distinct, isolated first and second indentation regions, the forming step comprising transferring at least one fluid precursor from the contoured surface of the article (20) to distinct first and second portions of the substrate surface (28) while leaving an intervening portion of the substrate surface (28) free of the at least one precursor.

10. A method as in claim 9, wherein the transferring step involves transferring the at least one fluid precursor to the first and second portions of the substrate surface (28) simultaneously.

11. A method as in claim 1, comprising:
positioning the article (20) proximate the substrate surface (28) and applying, to the first region (34) proximate the substrate surface (28) via capillary action involving the article (20), the species (38) which is a chemically active agent coupled to a particulate carrier; and
allowing a chemical reaction involving the chemically active agent to take place at the first region (34) proximate the substrate surface (28).

12. A method as in claim 11, wherein the chemically active agent is coupled to a particulate carrier that is applied, in a fluid carrier via capillary action involving the article (20), to the first region (34) proximate the substrate surface (28).

13. A method as in either of claims 11 or 12, wherein the particulate carrier comprises microbeads.

14. A method as in claim 1, comprising:
forming at the first region (34) proximate the substrate surface (28), in the pattern corresponding to the indentation pattern, a pattern of a biochemically active agent.

15. A method as in claim 14, further comprising:
allowing a biochemical interaction involving the biochemically active agent to take place proximate the first region (34) of the substrate surface (28), in a pattern corresponding to the indentation pattern and in an area including a portion having a lateral dimension of less than 1 mm, while leaving a second region proximate the substrate surface (28), contiguous with the first region (34), free of the biochemical interaction.

16. A method as in claim 15, wherein the biochemically active agent is a biological binding partner and the biochemical interaction involves biological binding between the biochemically active agent and a second biochemically active agent.

17. A method as in any of claims 14-16, wherein the biochemically active agent includes a polyamino acid tag and the forming step involves immobilizing the biochemically active agent at the first region (34) proximate the substrate surface (28) via coordination of the polyamino acid tag to a metal ion coordinated to a chelating agent immobilized at the substrate surface(28).

18. A method as in any of claims 14-17, wherein the biochemically active agent includes a polyamino acid tag and the substrate surface (28) includes immobilized chelating agents coordinating metal ions, the forming step comprising forming a pattern of the biochemically active agent coordinated to the metal ion via the polyamino acid tag at a first region proximate (34) the substrate surface (28).

19. A method as in any of claims 14-18, wherein the biochemically active agent is coupled to a particulate carrier and the forming step comprises forming the particulate carrier at a first region proximate (34) the substrate surface (28).

20. A method as in any preceding claim, **characterized in that** the first region (34) includes a portion having a lateral dimension of less than about 500 microns, and, preferably, less than about 100, 50 or 20 microns.

21. A method of creating a pattern of a species comprising:
providing a surface (148) carrying an essentially even distribution thereacross of chelating agents coordinating metal ions (148), **characterized in that** method involves applying to two discrete regions (142, 144) at the surface (148), a species that is a biologically active agent, while leaving a region intervening the two discrete regions free of the biologically active agent.

22. A method as in claim 21, involving providing the species in a fluid carrier, positioning the fluid carrier at the two discrete regions (142, 144) proximate the surface (148) and allowing the fluid carrier to dissipate.

23. A method as in any of claims 21-22 involving transferring the species from an article (136) having a contoured surface including at least one indentation (138) defining a pattern to a first discrete region proximate the surface (148) in a pattern corresponding to the indentation pattern while leaving a second region proximate the surface, (148) contiguous with the first region, free of the species.

24. A method as in any of claims 21-23, involving transferring at least one species from a contoured surface of an article (136) to at least first and second distinct isolated portions of the surface (148) separated by an intervening region of the substrate surface (148) that remains free of the species.

25. A method as in any of claims 21-24, involving applying the species essentially instantaneously to a first discrete region proximate the substrate surface (148) in a pattern corresponding to the indentation pattern of an article (136).

26. A method as in any of claims 21-25, involving forming a pattern of species at the first and second portions proximate the substrate surface (148) simultaneously.

27. A method as in any of claims 21-26 involving positioning a contoured surface of an article (136) proximate the substrate surface (148) and applying, to a first discrete region proximate the substrate surface (148) via capillary action involving the article (136), the species.

28. A method as in any of claims 21-27, involving positioning a contoured surface of an article (136) proximate the substrate surface (148) so as to create at least one channel defined by the at least one indentation at a first discrete region of the substrate surface and introducing the species into the channel.

29. A method as in claim 28, involving allowing the species to be introduced into the at least one channel via capillary action involving the at least one channel.

30. A method as in any of claims 21-29 involving forming a pattern of the species at a first discrete region in an area including a portion having a lateral dimension of less than about one millimeter.

31. A method as in any of claims 21-30, involving forming a pattern of the species at a first discrete region proximate the substrate surface (148) in an area including a portion having a lateral dimension of less than about 500 microns, and preferably, less than about 100, 50 or 20 microns.

32. A method as in claim 28, involving allowing the species to be introduced into the at least one channel via a vacuum created within the channels.

## Patentansprüche

1. Ein Verfahren zur Erzeugung eines Musters einer Spezies (38) in einem ersten Bereich (34) in der Nähe einer Substratoberfläche (28), wobei das Muster einen Bereich mit einer lateralen Erstreckung von weniger als 1mm aufweist, während ein zweiter Bereich in der Nähe der Substratoberfläche (28) und an den ersten Bereich (34) angrenzend, frei von dieser Art von Muster belassen wird, unter Verwendung eines Gegenstandes (20) mit einer konturierten Oberfläche (22) einschließlich wenigstens einer Vertiefung (24), die ein Muster darstellt,
**dadurch gekennzeichnet, daß** das verfahren die Erzeugung eines Musters einer Spezies (38) im ersten Bereich (34) in der Nähe der Substratoberfläche (28) in einer Art umfaßt, daß diese mit dem Vertiefungsmuster des Gegenstandes (20) übereinstimmt und in dieses hineinpaßt.

2. Ein Verfahren gemäß Anspruch 1, das folgendes umfaßt:
die Erzeugung einer Spezies, die eine flüssige Vorstufe einer anorganischen Spezies darstellt, im ersten Bereich (34) in der Nähe der Substratoberfläche (28) mit einem Muster, das dem Vertiefungsmuster entspricht: and
das Aushärtenlassen der flüssigen Vorstufe im ersten Bereich (34) in der Nähe der Substratoberfläche (28) zu einer anorganischen Spezies mit einem Muster, das dem Vertiefungsmuster entspricht.

3. Ein Verfahren gemäß Anspruch 1, das folgendes umfaßt:
die Erzeugung einer Suspension von Teilchen in einem flüssigen Träger im ersten Bereich (34) in der Nähe der Substratoberfläche (28) mit einem Muster, das dem Vertiefungsmuster entspricht: und
das Verdunstenlassen des flüssigen Trägers, der dabei die Teilchen im ersten Bereich (34) in der Nähe der Substratoberfläche (28) mit einem Muster zuruckläßt, das dem Vertiefungsmuster entspricht.

4. Das Verfahren gemäß Anspruch 3, wobei die Teilchen Mikrowülste bilden.

5. Ein Verfahren gemäß Anspruch 1, das folgendes umfaßt:
die Herstellung eines Agens, das geeignet ist, die Abscheidung eines Metalls zu unterstützen, im ersten Bereich (34) in der Nähe der Substratoberfläche (28) mit einem Muster, das dem Vertiefungsmuster entspricht.

6. Ein Verfahren gemäß Anspruch 5, wobei das Agens auf der Substratoberfläche (28) in einem flüssigen Träger erzeugt wird und wobei das Verfahren das Verdunstenlassen des flüssigen Trägers vorsieht, der dabei die chemisch aktive Substanz im ersten Bereich (34) in der Nähe der Substratoberfläche (28) zurückläßt.

7. Ein Verfahren gemäß Anspruch 1, das folgendes umfaßt:
die Herstellung einer flüssigen Vorstufe der Spezies im ersten Bereich (34) in der Nähe der Substratoberfläche (28) mit einem Muster, das dem Vertiefungsmuster entspricht, durch die Überführung der flüssigen Vorstufe von der konturierten Oberfläche des Gegenstandes (20) in den ersten Bereich (34) der Substratoberfläche (28) in dem Muster: und
das Aushärtenlassen der flüssigen Vorstufe im ersten Bereich (34) in der Nähe der Substratoberfläche (28) in einem Muster, das dem Vertiefungsmuster entspricht.

8. Ein Verfahren gemäß Anspruch 7, das vorsieht, daß die flussige Vorstufe im wesentlichen sofort von der konturierten Oberfläche des Gegenstandes (20) in den ersten Bereich (34) der Substratoberfläche (28) überführt wird.

9. Ein Verfahren gemäß Anspruch 8, wobei das Muster unterschiedliche, räumlich deutlich voneinander getrennte erste und zweite Vertiefungsbereiche aufweist und wobei der Herstellungsschritt die Überführung wenigstens einer flüssigen Vorstufe von der konturierten Oberfläche des Gegenstandes (20) in die getrennten ersten und zweiten Bereiche der Substratoberfläche (28) umfaßt, während ein dazwischen liegender Bereich der Substratoberfläche (28) frei von der wenigstens einen flüssigen Vorstufe verbleibt.

10. Ein Verfahren gewäß Anspruch 9, wobei der Schritt der Überführung die gleichzeitige Überführung der wenigstens einen flüssigen Vorstufe in die ersten und zweiten Bereiche der Substratoberfläche (28) vorsieht.

11. Ein Verfahren gemäß Anspruch 1, das folgendes umfaßt:
die Positionierung des Gegenstandes (20) in der Nähe der Substratoberfläche (28) und das Aufbringen der Spezies (38), die aus einer chemisch aktiven Substanz besteht, die mit einem korpuskularen Träger verbunden ist, in den ersten Bereich (34) in der Nähe der Substratoberfläche (28) mittels Kapillarwirkung unter Verwendung des Gegenstandes (20) : und
daß die chemische Reaktion unter Einbeziehung der chemisch aktiven Substanz im ersten Bereich (34) in der Nähe der Substratoberfläche (28) stattfinden kann.

12. Ein Verfahren gemäß Anspruch 11, wobei die chemisch aktive Substanz mit einem korpuskularen Träger verbunden ist, der in einem flüssigen Träger mittels Kapillarwirkung unter verwendung des Gegenstandes (20) im ersten Bereich (34) in der Nähe der Substratoberfläche (28) aufgebracht wird.

13. Ein Verfahren gemäß einem der Ansprüche 11 oder 12, wobei der korpuskulare Träger Mikrowülste aufweist.

14. Ein Verfahren gemäß Anspruch 1, das folgendes umfaßt:
die Bildung einer biochemisch aktiven Substanz im ersten Bereich (34) in der Nähe der Substratoberfläche (28) mit einem Muster, das dem Vertiefungsmuster entspricht.

15. Ein Verfahren gemäß Anspruch 15, das weiterhin umfaßt.:
daß eine biochemische Wechselwirkung unter Einbeziehung der biochemisch aktiven Substanz im ersten Bereich (34) in der Nähe der Substratoberfläche (28) stattfinden kann, mit einem Muster, das dem Vertiefungsmuster entspricht und in einem Bereich, der einen Teilbereich umfaßt, der eine laterale Erstreckung von weniger als 1mm aufweist, während ein zweiter Bereich in der Nähe der Substratoberfläche (28) und an den ersten Bereich (34) angrenzend von der biochemischen Wechselwirkung freigehalten wird.

16. Ein Verfahren gemäß Anspruch 15, wobei die biochemisch aktive Substanz aus einem biologischen Binder besteht und die biochemische Wechselwirkung das biologische Verbinden zwischen der biochemisch aktiven Substanz und einer zweiten biochemisch aktiven Substanz umfaßt.

17. Ein Verfahren gemäß einem der Ansprüche 14 **-** 16, wobei die biochemisch aktive Substanz einen Polyaminosäure-Marker umfaßt und der Herstellungsschritt die Immobilisierung der biochemisch aktiven Substanz im ersten Bereich (34) in der Nähe der Substratoberfläche (28) durch die Reaktion des Polyaminosäure-Markers mit einem Metall-Ion umfaßt, das mit einem chelatbildenden Agens verbunden ist, das an der Substratoberfläche (28) immobilisiert ist.

18. Ein Verfahren gemäß einem der Ansprüche 14 - 17, wobei die biochemisch aktive Substanz einen Polyaminosäure-Marker enthält und. die Substratoberfläche (28) Metall-Ion aufweist, die mit einem chelatbildenden Agens verbunden sind, wobei der Herstellungsschritt die Erzeugung eines Musters der biochemisch aktiven Substanz, die durch die Reaktion des Polyaminosäure-Markers mit dem Metall-Ion verbunden ist, in einem ersten Bereich (34) in der Nähe der Substratoberfläche (28) umfaßt.

19. Ein Verfahren gemäß einem der Ansprüche 14 - 18, wobei die biochemisch aktive Substanz mit einem korpuskularen Träger verbunden ist und wobei der Herstellungsschritt das Aufbringen des korpuskularen Trägers im ersten Bereich (34) in der Nähe der Substratoberfläche (28) umfaßt.

20. Ein Verfahren gemäß einem der vorangehenden Ansprüche, **dadurch gekennzeichnet, daß** der erste Bereich (34) einen Bereich umfaßt, der eine laterale Erstreckung von weniger als 500 Mikrometern und vorzugsweise von weniger als 100, 50 oder 20 Mikrometern aufweist.

21. Ein Verfahren zur Erzeugung eines Musters einer Spezies, das folgendes umfaßt:
die Erzeugung einer Oberfläche (148) mit einer im wesentlichen gleichmäßigen Verteilung von Metall-Ionen (148), die mit einem chelatbildenden Agens verbunden sind, **dadurch gekennzeichnet, daß** das Verfahren das Aufbringen einer Spezies aus einer biologisch aktiven Substanz auf zwei verschiedene Bereiche (142, 148) an der Oberfläche (148) umfaßt, während der zwischen diesen beiden verschiedenen Bereichen befindliche Bereich von der biologisch aktiven Substanz freigehalten wird.

22. Ein Verfahren gemäß Anspruch 21, wobei die Spezies in einem flüssigen Träger aufgebracht wird, wobei der flüssige Träger auf den zwei verschiedenen Bereichen (142, 148) in der Nähe der oberfläche (148) positioniert wird und wobei der flüssige Träger verdunstet.

23. Ein Verfahren gemäß einem der Ansprüche 21-22, das die Überführung der Spezies von einem Gegenstand (136) mit einer konturierten Oberfläche einschließlich wenigstens einer Vertiefung (138), die ein Muster darstellt, zu einem ersten bestimmten Bereich in der Nähe der Oberfläche (148) in einem Muster umfaßt, das mit dem Vertiefungsmuster übereinstimmt, während ein zweiter Bereich in der Nähe der Oberfläche (148), der an den ersten Bereich angrenzt, von der Spezies freigehalten wird.

24. Ein Verfahren gemäß einem der Ansprüche 21-23, das die Überführung wenigstens einer Spezies von einer konturierten Oberfläche eines Gegenstandes (136) zu wenigstens einem ersten und zweiten getrennten Bereich der Oberfläche (148) umfaßt, wobei diese Bereiche durch einen dazwischen liegenden Bereich der Oberfläche (148) getrennt sind, der von der Spezies freigehalten wird.

25. Ein Verfahren gemäß einem der Ansprüche 21-24, das das im wesentlichen sofortige Aufbringen der Spezies auf einen ersten bestimmten Bereich in der Nähe der Oberfläche (148) mit einem Muster umfaßt, das mit dem Vertiefungsmuster des Gegenstandes (136) übereinstimmt.

26. Ein Verfahren gemäß einem der Ansprüche 21-25, das die gleichzeitige Erzeugung eines Musters der Spezies in den ersten und zweiten Bereichen in der Nähe der Substratoberfläche (148) vorsieht.

27. Ein Verfahren gemäß einem der Ansprüche 21-26, das die Positionierung der konturierten Oberfläche eines Gegenstandes (136) in der Nähe der Substratoberfläche (148) und das Aufbringen der Spezies in einen ersten begrenzten Bereich in der Nähe der Substratoberfläche (148) mittels Kapillarwirkung unter Verwendung des Gegenstandes (136) umfaßt.

28. Ein Verfahren gemäß einem der Ansprüche 21-27, das die Positionierung der konturierten Oberfläche eines Gegenstandes (136) in der Nähe der Substratoberfläche (148) derart umfaßt, daß wenigstens ein Kanal von der wenigstens einen Vertiefung in einem ersten begrenzten Bereich der Substratoberfläche (148) gebildet wird, sowie das Einbringen der Spezies in diesen Kanal.

29. Ein Verfahren gemäß Anspruch 28, wobei die Spezies in den wenigstens einen Kanal mittels Kapillarwirkung unter verwendung des wenigstens einen Kanals eingebracht wird.

30. Ein Verfahren gemäß einem der Ansprüche 21-29, das die Erzeugung eines Musters der Spezies in einem ersten begrenzten Bereich einer Fläche umfaßt, die einen Teil mit einer lateralen Erstreckung von weniger als etwa einem Millimeter aufweist.

31. Ein verfahren gemäß einem der Ansprüche 21-30, das die Erzeugung eines Musters der Spezies in der Nähe der Substratoberfläche (148) in einer Fläche umfaßt, die einen Teil mit einer lateralen Erstreckung von weniger als etwa 500 Mikrometern und vorzugsweise von weniger als etwa 100, 50 oder 20 Mikrometern aufweist.

32. Ein verfahren gemäß Anspruch 28, wobei die Spezies in den wenigstens einen Kanal mittels eines in den Kanälen erzeugten Vakuums eingebracht wird.

## Revendications

1. Une méthode pour la création d'un motif d'espèces (38) dans une première région (34) à proximité de la surface (28) d'un substrat, ce motif comportant une portion présentant une dimension latérale inférieure à 1 mm, tout en laissant une seconde région à proximité de la surface (28) du substrat contiguë à la première région (34), exempte de l'espèce, en utilisant un article (20) présentant une surface contourée (définie par un contour) (22) comprenant au moins une indentation (24) définissant un motif,
**caractérisée en ce que** cette méthode comporte la création d'un motif de l'espèce (38) dans la première région (34) à proximité de la surface (28) du substrat d'une manière correspondant à et conforme au motif d'indentation de l'article (20).

2. Une méthode selon la revendication 1 consistant à :
former dans la première région (34) à proximité de la surface (28) du substrat, conformément à un motif correspondant au motif de l'indentation, une espèce qui est un précurseur fluidique d'une espèce inorganique ; et
permettre au précurseur fluidique de se durcir dans la première région (34) de la surface (28) du substrat sous forme d'une espèce inorganique conformément à un motif correspondant au motif de l'indentation.

3. Une méthode selon la revendication 1 consistant à :
former dans la première région (34) à proximité de la surface (28) du substrat, conformément à un motif correspondant au motif de l'indentation, une suspension de particules dans un véhicule fluide ; et
permettre au véhicule fluide de se dissiper en déposant ainsi les particules dans la première région (34) de la surface (28) du substrat conformément à un motif correspondant au motif de l'indentation.

4. Une méthode selon la revendication 3, dans laquelle les particules sont des microsphères.

5. Une méthode selon la revendication 1, consistant à :
former dans la première région (34) à proximité de la surface (28) du substrat, conformément à un motif correspondant au motif de l'indentation, un agent susceptible de déterminer le dépôt d'un métal.

6. Une méthode selon la revendication 5, dans laquelle l'agent est formé dans la surface (28) du substrat dans un véhicule fluide, cette méthode consistant à permettre au véhicule fluide de se dissiper en déposant ainsi l'agent chimiquement actif dans la première région (34) de la surface (28) du substrat.

7. Une méthode selon la revendication 1, consistant à :
former dans la première région (34) à proximité de la surface (28) du substrat, conformément à un motif correspondant au motif de l'indentation, un précurseur fluidique de l'espèce en transférant le précurseur fluidique depuis la surface contourée de l'article (20) à la première région (34) de la surface (28) du substrat dans le motif ; et
permettre au précurseur fluidique de se durcir dans la première région (34) de la surface (28) du substrat conformément à un motif correspondant au motif de l'indentation.

8. Une méthode selon la revendication 7, consistant à transférer le précurseur fluidique de manière pratiquement instantanée depuis la surface contourée de l'article (20) jusqu'à la première région (34) de la surface (28) du substrat.

9. Une méthode selon la revendication 8, dans laquelle le motif comprend une première et une seconde région d'indentation isolées distinctes, l'étape de formation consistant à transférer au moins un précurseur fluidique depuis la surface contourée de l'article (20), jusqu'à la première et la seconde portion distinctes de la surface (28) du substrat tout en laissant une portion intervenante de la surface (28) du substrat exempte dudit précurseur.

10. Une méthode selon la revendication 9, dans laquelle l'étape de transfert consiste à transférer au moins un précurseur fluidique simultanément jusqu'aux première et seconde portions de la surface (28) du substrat.

11. Une méthode selon la revendication 1, comprenant les étapes consistant à :
placer l'article (20) à proximité de la surface (28) du substrat et appliquer à la première région (34) à proximité de la surface (28) du substrat, par action capillaire impliquant l'article (20), l'espèce (38) qui est un agent chimiquement actif couplé à un véhicule particulaire ; et
permettre à une réaction chimique impliquant l'agent chimiquement actif de se produire dans la première région (34) à proximité de la surface (28) du substrat.

12. Une méthode selon la revendication 11, dans laquelle l'agent chimiquement actif est couplé à un véhicule particulaire qui est appliqué dans un véhicule fluidique par action capillaire impliquant l'article (20) à la première région (34) à proximité de la surface (28) du substrat.

13. Une méthode selon l'une des revendications 11 ou 12, dans laquelle le véhicule particulaire consiste en microsphères.

14. Une méthode selon la revendication 1, consistant à former dans la première région (34) à proximité de la surface (28) du substrat, conformément au motif correspondant au motif de l'indentation, un motif d'un agent bio-chimiquement actif.

15. Une méthode selon la revendication 14, consistant au surplus à permettre à une interaction biochimique faisant intervenir l'agent actif bio-chimiquement de se produire à proximité de la première région (34) de la surface (28) du substrat, conformément à un motif correspondant au motif de l'indentation et dans une zone incluant une portion ayant une dimension latérale inférieure à 1 mm, tout en laissant une seconde région à proximité de la surface (28) du substrat, contiguë à la première région (34) exempte de l'interaction biochimique.

16. Une méthode selon la revendication 15, dans laquelle l'agent bio-chimiquement actif est un partenaire de fixation biologique et l'interaction biologique fait intervenir une liaison biologique entre l'agent bio-chimiquement actif et un second agent bio-chimiquement actif.

17. Une méthode selon l'une quelconque des revendications 14-16, dans laquelle l'agent bio-chimiquement actif est un acide poly-aminé marqueur et l'étape de formation implique l'immobilisation de l'agent bio-chimiquement actif dans la première région (34) à proximité de la surface (28) du substrat par coordination du poly-aminoacide marqueur avec un ion métallique par coordination de manière à créer un agent chélatant immobilisé sur la surface (28) du substrat.

18. Une méthode selon l'une quelconque des revendications 14-17, dans laquelle l'agent bio-chimiquement actif est un poly-aminoacide marqueur et la surface (28) du substrat consiste en des agents chélatants immobilisés coordonnant des ions métalliques, l'étape de formation consistant à former un motif de l'agent bio-chimiquement actif coordonné avec l'ion métallique par l'intermédiaire du poly-aminoacide marqueur dans une première région à proximité (34) de la surface (28) du substrat.

19. Une méthode selon l'une quelconque des revendications 14-18, dans laquelle l'agent bio-chimiquement actif est couplé avec un véhicule particulaire et l'étape de formation consiste à former le véhicule particulaire dans une première région (34) à proximité de la surface (28) du substrat.

20. Une méthode selon l'une quelconque des revendications précédentes, **caractérisée en ce que** la première région (34) englobe une portion présentant une dimension latérale inférieure à environ 500 microns et de préférence inférieure à environ 100, 50 ou 20 microns.

21. Une méthode pour la création d'un motif d'une espèce consistant à créer une surface (148) portant une répartition essentiellement régulière d'agents chélatants constitués par des ions métalliques de coordination (148), **caractérisée en ce que** ladite méthode consiste à appliquer dans deux régions individuelles (142, 144) de la surface (148) une espèce constituée par un agent biologiquement actif, tout en laissant une région faisant intervenir les deux régions individuelles exempte de l'agent bio-chimiquement actif.

22. Une méthode selon la revendication 21, consistant à apporter l'espèce dans un véhicule fluide, à placer le véhicule fluide dans les deux régions individuelles (142, 144) à proximité de la surface (148) et à permettre au véhicule fluide de se dissiper.

23. Une méthode selon l'une quelconque des revendications 21 et 22, consistant à transférer l'espèce depuis un article (136) présentant une surface contourée incluant au moins une indentation (138) définissant un motif dans une première région individuelle à proximité de la surface (148) conformément à un motif correspondant au motif de l'indentation tout en laissant une seconde région à proximité de la surface (148) contiguë à la première région, exempte de l'espèce.

24. Une méthode selon l'une quelconque des revendications 21 à 23, consistant à transférer au moins une espèce depuis une surface contourée d'un article (136) vers au moins l'une d'une première et d'une seconde portions isolées distinctes de la surface (148) séparées par une région intervenante de la surface (148) du substrat qui demeure exempte de l'espèce.

25. Une méthode selon l'une quelconque des revendications 21 à 24, consistant à appliquer l'espèce de manière pratiquement instantanée à une première région individuelle à proximité de la surface (148) du substrat conformément à un motif correspondant au motif de l'indentation d'un article (136).

26. Une méthode selon l'une quelconque des revendications 21 à 25, consistant à former un motif de l'espèce simultanément sur la première et la seconde portions à proximité de la surface (148) du substrat.

27. Une méthode selon l'une quelconque des revendications 21 à 26, consistant à placer une surface contourée d'un article (136) à proximité de la surface (148) du substrat et à appliquer l'espèce à une première région individuelle à proximité de la surface (148) du substrat par une action capillaire impliquant l'article (136).

28. Une méthode selon l'une quelconque des revendications 21 à 27, consistant à placer une surface contourée d'un article (136) à proximité de la surface (148) du substrat de manière à créer au moins un canal défini par ladite indentation sur une première région individuelle de la surface du substrat et à introduire l'espèce dans le canal.

29. Une méthode selon la revendication 28, consistant à permettre à l'espèce d'être introduite dans ledit canal par une action capillaire impliquant ledit canal.

30. Une méthode selon l'une quelconque des revendications 21 à 29, consistant à former un motif de l'espèce dans une première région individuelle dans une zone incluant une portion ayant une dimension latérale d'au moins environ 1 mm.

31. Une méthode selon l'une quelconque des revendications 21 à 30, consistant à former un motif de l'espèce dans une première région individuelle à proximité de la surface (148) du substrat dans une zone incluant une portion ayant une dimension latérale inférieure à environ 500 microns et de préférence inférieure à environ 100, 50 ou 20 microns.

32. Une méthode selon la revendication 28, consistant à permettre à l'espèce d'être introduite dans au moins un canal par un vide créé entre les canaux.
